# EUROPEAN PATENT SPECIFICATION

(11) **EP 4 510 953 B1**
(45) Date of publication and mention of the grant of the patent: **04.03.2026**
(21) Application number: 23721839.1
(22) Date of filing: 19.04.2023
(51) Int. Cl.: A61B 17/435, A61B 90/00

(54) **SYSTEM FOR EMBRYO TRANSFER**
SYSTEM FÜR EMBRYOTRANSFER
SYSTÈME DE TRANSFERT D'EMBRYONS

(30) Priority: 19.04.2022 EP 22382364; 14.11.2022 ES 202231892 U; 14.11.2022 EP 22383099
(43) Date of publication of application: 26.02.2025
(73) Proprietor: Premium Fertility S.L., 46980 Paterna (Valencia) (ES)
(72) Inventor: SANTAMARÍA COSTA, Xavier, 46980 Paterna (Valencia) (ES); SIMÓN VALLÉS, Carlos, 46980 Paterna (Valencia) (ES); FREITAS, Rhys, San Diego, California 92127 (US); BADAL REGAS, Ari, San Diego, California 92127 (US); HUNT, David, S., San Diego, California 92127 (US)
(74) Representative: Hoffmann Eitle
(86) International application number: PCT/EP2023/060081
(87) International publication number: WO 2023/203052

(56) References cited:
- WO-A1-2019/243884
- WO-A1-99/37348
- US-A- 5 496 272
- US-A- 5 746 694
- US-A- 6 010 448
- US-A1- 2004 122 286
- US-A1- 2014 276 026
- US-A1- 2015 342 642

## Description

### TECHNICAL FIELD

The present disclosure relates to systems and methods for transferring a fertilized egg or embryo to the endometrial epithelium. The disclosure also relates to a device for guiding such systems and for engaging a speculum.

### BACKGROUND

Human In Vitro Fertilization (IVF) and Embryo Transfer (ET), first successfully performed in 1978, has become a widely practiced procedure to treat infertile couples who have failed with more conventional methods of therapy such as superovulation and intrauterine insemination. The most common indications for IVF and related procedures, such as Gamete In Vitro Fertilization or Gamete Intra-Fallopian Transfer (GIFT) which includes women having blocked or damaged fallopian tubes and includes low sperm and/or egg quality. Related factors include age of the female, and the degree of endometrial receptivity. The procedure may also be used in cases of severe male factor where direct (intracytoplasmic) injection of sperm is an option.

The IVF/ET procedure typically involves the hormonal stimulation of the female to first suppress her ability to ovulate on her own, then stimulate development of follicles in the ovaries with a fertility medication. The mature eggs are removed from the ovary transvaginally using a needle, preferably guided under ultrasound. Following harvesting of the eggs, the eggs are identified and sorted with regard to maturity, and then placed with a sperm sample from the male. Approximately 24 hours after fertilization, the eggs are examined to confirm fertilization, which occurs in approximately 65% to 85% of the eggs harvested.

After a short development period, the embryos are transferred, along with a volume of fluid, to the uterus using a delivery catheter. The delivery catheter is usually made of a soft plastic material to avoid damage to the endometrium. There are many potential difficulties in achieving a successful implantation. Because of the soft nature of the standard delivery catheter, in a number of cases, the tip of the catheter may bend back on itself or curve away from the fundus of the uterus. The tip may also accidentally pass between the layer of the endometrium and myometrium. Conversely, a stiffer catheter increases the risk of trauma to the uterus or cervix, with the latter possibly leading to the release of prostaglandins and expulsion of the eggs from the endometrium.

One particular difficulty in achieving successful implantation is the difficulty the surgeon has in visualizing the uterus and the endometrium into which the embryo is implanted. In particular, mucus and other bodily fluids may make it difficult for the surgeon to determine whether the endometrial epithelium has been reached.

It has also been observed that the fertilized egg can be expelled from the endometrial epithelium or dislodged after the transfer procedure, increasing the risk of a failed procedure. Similarly, the fertilized egg may remain stuck to the transfer apparatus even after the medium containing the fertilized egg is injected into the endometrial epithelium.

Further, a wide variety of speculums are used of different shapes and sizes, making it difficult for an embryo transfer apparatus to spatially lock to the anatomy of the patient in order to increase the accuracy of the embryo delivery.

There is therefore a need for improved embryo transfer apparatuses with an increased reliability and for a way of locking such embryo transfer apparatuses to a variety of speculum shapes.

The following documents disclose prior art devices: WO 99/37348 A1, US 2004/122286 A1, WO 2019/243884 A1, US 6 010 448 A, US 5 496 272 A, US 2015/342642 A1, US 2014/276026 A1, US 5 746 694 A.

### SUMMARY OF THE INVENTION

According to a first aspect of the disclosure, there is provided a system for delivering a fertilized egg or embryo into a maternal uterine endometrium in humans or any other mammalian species, comprising: a body having a distal end suitable for penetrating the endometrial epithelium; a plunger slidably received in a lumen of the body; an actuator operable advance the plunger to expel a fertilized egg from the lumen of the body; a first injectable medium; and a second injectable medium for containing the fertilized egg; wherein the first injectable medium has a higher viscosity than the second injectable medium.

According to a second aspect of the disclosure, there is provided a method of loading a system for delivering a fertilized egg or embryo into a maternal uterine endometrium in humans or any other mammalian species, the system comprising: a body having a distal end suitable for penetrating the endometrial epithelium; a plunger slidably received in a lumen of the body; and an actuator operable to advance the plunger to expel a fertilized egg from the lumen of the body; wherein the method comprises: drawing a first injectable medium into the lumen; and subsequently drawing a second injectable medium containing a fertilized egg into the lumen; wherein the first injectable medium has a higher viscosity than the second injectable medium.

According to the invention and as defined in claim 1, there is provided an apparatus suitable for delivering a fertilized egg or embryo into a maternal uterine endometrium in humans or any other mammalian species, comprising a body configured to fit within a lumen of the female reproductive system, the body comprising: one or more lumens extending from a proximal end of the body to a distal portion of the body and having a distal opening at the distal portion of the body, wherein a first lumen of the one or more lumens slidably receives an inner body having a distal end suitable for penetrating the endometrial epithelium, the inner body comprising an inner lumen for receiving a fertilized egg; an actuator operable to expel the fertilized egg from the inner lumen; and a covering layer at least partially covering the distal opening of at least one lumen of the one or more lumens. The covering layer at least partially covers the distal opening of the first lumen and the inner body is configured to perforate through the covering layer when the inner body is advanced out from the distal opening of the body.

According to a fourth aspect of the disclosure, there is provided a device for engaging a speculum, comprising: two or more expandable engaging elements for outwardly engaging a speculum to fix the device to the speculum, wherein the two or more expandable engaging elements comprise a plurality of expanded configurations for engaging a plurality of speculum sizes; and a guide for guiding an embryo delivery device between the expandable engaging elements and through the speculum when the device is fixed to the speculum.

According to a fifth aspect of the disclosure, there is provided an apparatus suitable for delivering a fertilized egg or embryo into a maternal uterine endometrium in humans or any other mammalian species, comprising a body configured to fit within a lumen of the female reproductive system, the body comprising: a lumen extending from a proximal end of the body to a distal portion of the body and having a distal opening at the distal portion of the body, the lumen slidably receiving an inner body having a distal end suitable for penetrating the endometrial epithelium; the apparatus further comprising: a first actuator operable to advance the inner body out from the distal opening of the body; and a second actuator operable to expel a fertilized egg from an inner lumen of the inner body; wherein the inner body comprises a hydrophobic coating formed on a distal portion of the inner body.

### BRIEF DESCRIPTION OF THE DRAWINGS

To enable better understanding of the present disclosure, and to show how the same may be carried into effect, reference will now be made, by way of example only, to the accompanying schematic drawings, in which:
Fig. 1A shows a perspective view of an apparatus suitable for delivering a fertilized egg or embryo into a maternal uterine endometrium according to one or more embodiments shown and described herein.
Fig. 1B shows a perspective view of another apparatus suitable for delivering a fertilized egg or embryo into a maternal uterine endometrium according to one or more embodiments of the invention.
Fig. 1C shows a perspective view of another apparatus suitable for delivering a fertilized egg or embryo into a maternal uterine endometrium according to one or more embodiments shown and described herein.
Fig. 1D shows a side view of a distal portion of the apparatus shown in Fig. 1C.
Fig. 1E shows a front view of the apparatus shown in Fig. 1C.
Fig. 1F shows a perspective view of another apparatus suitable for delivering a fertilized egg or embryo into a maternal uterine endometrium according to one or more embodiments shown and described herein.
Fig. 1G shows a perspective view of another apparatus suitable for delivering a fertilized egg or embryo into a maternal uterine endometrium according to one or more embodiments shown and described herein.
Fig. 1H shows a perspective view of another apparatus suitable for delivering a fertilized egg or embryo into a maternal uterine endometrium according to one or more embodiments shown and described herein.
Fig. 1J shows a schematic side view of a body for an apparatus according to one or more embodiments shown and described herein.
Fig. 1K shows a schematic side view of a stylet for an apparatus according to one or more embodiments shown and described herein.
Fig. 1L shows a schematic cross-sectional side view of the stylet of Fig. 1K inserted within a body for an apparatus according to one or more embodiments.
Fig. 2A shows a schematic perspective view of a system for delivering a fertilized egg or embryo into a maternal uterine endometrium in a first configuration according to one or more embodiments shown and described herein.
Figs. 2B to 2D show schematic perspective views of the system of Fig. 2A in second to fourth configurations.
Fig. 2E shows a schematic view of a first, second and third medium situated in the endometrial epithelium.
Fig. 3 shows a schematic view of a bevelled tip according to one or more embodiments shown and described herein.
Fig. 4 shows a diagram of an indicating device according to one or more embodiments shown and described herein.
Fig. 5 shows a diagram of a computer that may be used to control the apparatus according to one or more embodiments shown and described herein.
Fig. 6A shows a cross-sectional perspective view of a connector according to one or more embodiments shown and described herein.
Fig. 6B shows a close-up view of a part of the connector shown in Fig. 6A.
Fig. 6C shows a perspective view of a proximal end of a connector according to one or more embodiments shown and described herein.
Fig. 6D shows a perspective view of a distal end of a motion controller according to one or more embodiments shown and described herein.
Fig. 6E shows an exploded view of the connector shown in Fig. 6A.
Fig. 6F shows a perspective view of a connection between a body connector and a distal end of a motion controller according to one or more embodiments shown and described herein.
Fig. 6G shows a perspective view of a proximal portion of a motion controller according to one or more embodiments shown and described herein.
Fig. 6H shows a perspective view of a motion controller according to one or more embodiments shown and described herein.
Fig. 7A shows a cross-sectional side view of a device for engaging a speculum according to one or more embodiments shown and described herein.
Fig. 7B shows a cross-sectional side view of a device for engaging a speculum according to one or more embodiments shown and described herein.
Fig. 7C shows the device of Fig. 7B in a second configuration.
Fig. 8A shows a schematic perspective front view of a device for engaging a speculum in a first configuration according to one or more embodiments shown and described herein.
Fig. 8B shows a schematic perspective side view of the device of Fig. 8A in the first configuration.
Fig. 8C shows a schematic perspective front view of the device of Fig. 8A in a second configuration.
Fig. 8D shows a schematic perspective front view of the device of Fig. 8A in a third configuration.
Fig. 9A shows a side view of a steering mechanism according to some embodiments shown and described herein.
Fig. 9B shows a side view of another steering mechanism according to some embodiments shown and described herein.
Fig. 9C shows a top view of the steering mechanisms shown in Figs. 9A or 9B.
Fig. 9D shows a side view of a steering mechanism with a cable in a retracted position according to one or more embodiments shown and described herein.
Figs. 9E shows a perspective view of a third actuator according to some embodiments shown and described herein.
Fig. 9F shows a perspective view of the third actuator shown in Fig. 8E with the outer shell depicted as transparent.
Fig. 9G shows a perspective view of an inner element of the steering mechanism according to some embodiments shown and described herein.
Fig. 9H shows a perspective view of a steering mechanism according to some embodiments shown and described herein, with the outer shell illustrated as partially transparent.
Fig. 10 shows a perspective view of an apparatus according to one or more embodiments.
Fig. 11 shows a perspective schematic view of an apparatus according to one or more embodiments.
Figs. 12A and 12B show schematic side views of an apparatus according to one or more embodiments.
Fig. 13 shows a schematic side view of an apparatus according to one or more embodiments.
Fig. 14A shows a schematic top view of an apparatus according to one or more embodiments.
Fig. 14B shows a cross-sectional side view of the apparatus shown in Fig. 14A taken along the line A-A.

### DETAILED DESCRIPTION

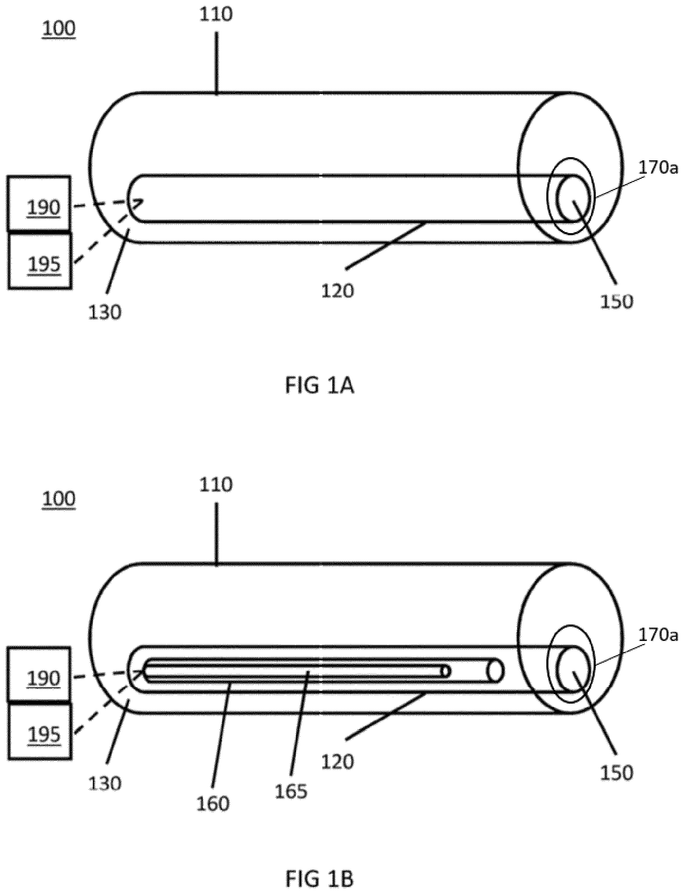

Fig. 1A shows a perspective view of an apparatus 100 suitable for delivering a fertilized egg or embryo into a maternal uterine endometrium, wherein the apparatus 100 comprises a body 110 (e.g. a catheter, also referred to as an outer body) configured to fit within a lumen of the female reproductive system. The body comprises one or more lumens including a lumen 120 extending from a proximal end 130 of the body 110 to a distal portion 140 of the body 110 and having a distal opening 150 at the distal portion 140 of the body. The lumen 120 is configured to slidably receive an inner body 160 having a distal end suitable for penetrating the endometrial epithelium. The apparatus 100 may also comprise a first actuator 190 operable to advance the inner body 160 out from the distal opening 150 of the body 110, and a second actuator 195 operable to expel a fertilized egg or embryo from the inner body.

The apparatus 100 may comprise a covering layer 170a at least partially covering the distal opening of one or more of the lumens (for example in the embodiment illustrated in Fig. 1A the covering layer 170a covers the distal opening 150 of lumen 120). The provision of a covering layer 170a inhibits mucus and other bodily fluids from entering the lumen 120 as the apparatus is advanced through the female reproductive system. This prevents components which are slidably received within one or more of the covered lumens (for example inner body 160 or a measurement assembly) from being exposed to the bodily fluids which may make them reusable when they are extractable from the apparatus 100. Furthermore, when the covering layer 170a is used for a lumen which contains a measurement assembly, it decreases the variability in measurements made by the measurement assembly to provide more reliable measurements for determining when the endometrial epithelium. For example, if the covering layer 170a is not provided, mucus and other bodily fluid may enter the lumen in an unpredictable manner and this may affect measurements made by the measurement assembly and therefore reduce the accuracy of determining when the endometrial epithelium has been reached. For example, when the measurement assembly comprises a capacitance sensor configured to make electrical contact with a distal portion of the inner body, it has been observed that the present of mucus in the lumen reduces the ability of the capacitance sensor to differentiate between the first state and the second state. Similarly, when the apparatus comprises a camera, mucus entering the lumen receiving the camera can cause the view of the camera to be partially or even completely obscured.

The dimensions of the body 110 are exaggerated in Fig. 1A - 1G for the purposes of clarity, whereas in reality the body 110 is a narrow, elongate body configured to extend into the uterus to the endometrial epithelium.

Fig. 1B shows an embodiment of the apparatus 100 of Fig. 1A, wherein the apparatus 100 further includes an inner body 160 having a distal end suitable for penetrating the endometrial epithelium. In this embodiment the inner body 160 is shown located in the lumen 120 of the body 110. A plunger 165 is housed within the inner body 160 and is configured to advance towards the distal end of the inner body 160 when actuated by the second actuator 195 and expel a fertilized egg which is located inside the inner body 160. The covering layer 170a at least partially covers the distal opening 150 and the inner body 160 is configured to perforate through the covering layer 170a when the inner body 160 is advanced out from the distal opening of the body, such that the inner body 160 can be advanced through the covering layer 170a and out of the distal opening 150 to penetrate the endometrial epithelium. As used herein, the term "perforate" is understood to mean to pierce, in order to create a hole in the covering layer 170a. In other words, prior to perforation the portion of the covering layer 170a to be perforated is free from holes, slits or gaps through which mucus or other fluids may enter.

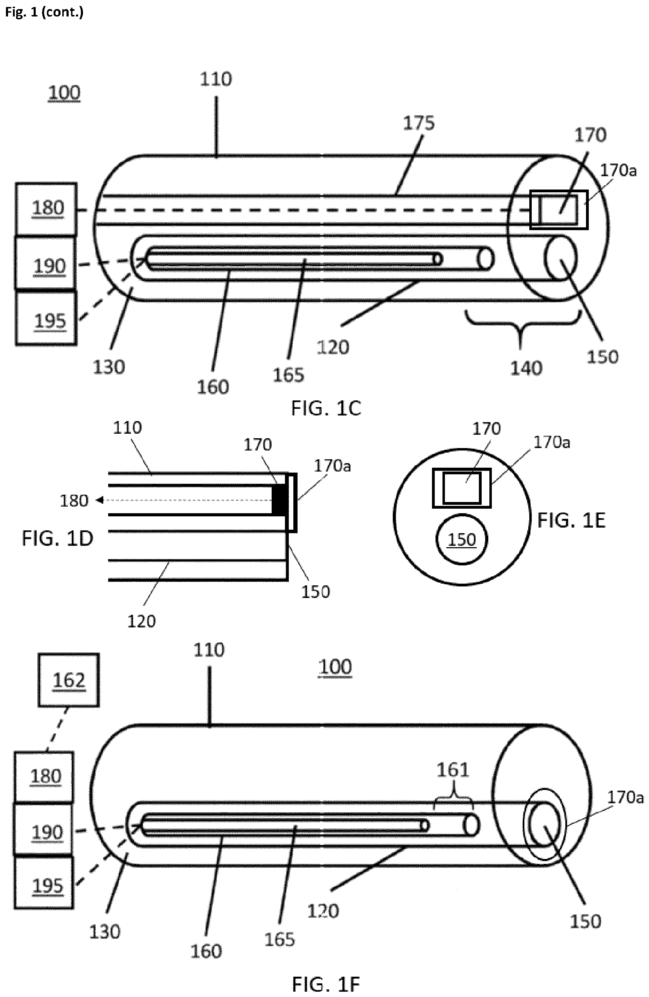

Fig. 1C shows a perspective view of another apparatus 100 suitable for delivering a fertilized egg or embryo into a maternal uterine endometrium, comprising a body 110 (e.g. a catheter, also referred to as an outer body) configured to fit within a lumen of the female reproductive system. Figs. 1D and 1E show side and front views of the distal portion of the apparatus 100 shown in Fig. 1C. The body 110 comprises one or more lumens including a lumen 120 extending from a proximal end 130 of the body 110 to a distal portion 140 of the body 110 and having a distal opening 150 at the distal portion 140 of the body. The lumen 120 is configured to slidably receive an inner body 160 having a distal end suitable for penetrating the endometrial epithelium. The apparatus 100 further comprises a measurement assembly comprising a measurement portion 170 disposed at the distal portion 140 of the body 110 (i.e. proximal to the distal opening 150). The measurement assembly 170 is configured to measure whether the apparatus is in a first state indicating the distance between the distal opening 150 of the body 110 and the endometrial epithelium is greater than a predetermined distance (i.e. in the direction that the inner body is advanced from the distal opening 150), or a second state indicating that the distance between the distal opening 150 of the body 110 and the endometrial epithelium is equal to or less than a predetermined distance. The apparatus 100 further comprises an indicating device 180 coupled to the measurement assembly 170 via a connection extending through a lumen 175 and configured to indicate that the measurement assembly 170 is in the first state or the second state. The measurement assembly may be removable from the body 110. In particular, the measurement portion 170 may be slidably received in a lumen of the body such that it may be extracted from the body 110 through the lumen, meaning the measurement assembly may be reusable. The inner body 160 and the measurement assembly may be received in the same or different lumens. The apparatus 100 also comprises a first actuator 190 operable to advance the inner body 160 out from the distal opening 150 of the body 110 by at least the predetermined distance, and a second actuator 195 operable to expel a fertilized egg from the inner body (for example to advance a plunger 165). The covering layer 170a at least partially covers at least one of the distal openings of the lumens. When the covering layer 170a covers the distal opening of the lumen 175 and thus the measurement portion 170, the covering layer prevents exposure of the measurement portion 170 to mucus and increases its reusability when the measurement assembly is removable from the apparatus (i.e. in embodiments where the measurement assembly is not fixedly attached to the inner lumen 175 and this removable).

In some embodiments, the measurement portion 170 is a camera proximal to the opening 150 and configured to view a portion of the endometrial epithelium.

The covering layer 170a preferably comprises a biocompatible transparent or semi-transparent film, and more preferably comprises or consists of polyether block amide (PEBAX), polyolefin, PVC, paraffin, cellulose and derivates, hyaluronic acid thin films, parylene C-films, collagen, gelatin, vegetable casings, alginates or composites or combinations thereof. Whilst the covering layer 170a would be expected to reduce the clarity of the image of the endometrial epithelium, it actually provides a clearer image than when mucus is allowed to enter into the lumen 175 and obscure the camera.

The predetermined distance may be, for example, less than 6mm. The inner body may be slidable to extend from the distal opening by any suitable distance, determined by the predetermined distance to the endometrial epithelium and the desired implantation depth of the embryo. For example, the inner body 160 may be slidable to extend from 0 to 6mm from the distal opening 150 of the body 110.

The body 110 may be an elongate body made of any suitable flexible and biocompatible material, such as PTFE, FEP, PEEK or other flexible lubricious material. Elongate body 110 and lumens may be formed, for example, by extrusion using known methods. The outer diameter of the body 110 may be between 1mm and 1.6mm, more preferably between 1.2mm and 1.4mm, and even more preferably 1.3mm. The total length of the body 110 may be any suitable length for insertion to a position proximal to the endometrial epithelium. For example, the body 110 may have a length of 400mm to 500mm, for example 450mm.

The inner body 160 may be made of any suitable biocompatible material such as PEEK and PI (Polyimide). Such materials enable the walls of the inner body 160 to be made relatively thin whilst maintaining the required rigidity of the inner body 160 for penetrating the endometrial epithelium. The inner body 160 may have any suitable outer diameter which slidably fits within the lumen 120 of the body 110. For example, the lumen 120 may have an inner diameter of greater than 0.45mm and the outer diameter of the inner body may be about 0.45mm.

The plunger 165 may also be made of materials such as PTFE, FEP, PEEK or other flexible lubricious material. The plunger 165 may have a suitable diameter which closely matches the inner diameter of the inner body 160 to effectively aspirate or expel fluid from the inner body 160. For example, the inner diameter of the inner body and the diameter of the plunger 165 may be about 0.28mm.

The measurement assembly may be any suitable measurement assembly that enables it to be determined whether the distance between the distal opening 150 of the body 110 and the endometrial epithelium is greater or less than the predetermined distance. It is noted that the measurement assembly may provide a qualitative measurement (for example the measurement assembly may transition between two states) or a quantitative measurement (for example actually measuring a parameter which indicates a value of the distance to the endometrial epithelium and comparing the parameter to a threshold value indicating the predetermined distance). The measuring portion 170 is the part of the measurement assembly which is configured to interact with the endometrial epithelium in order for the measurement assembly to make the determination and may comprise any suitable element or elements. The elements of the measuring portion 170 may be connected to components at the proximal end of the apparatus 100, such as components of the measurement assembly (not shown) and the indicating device 180 via connections extending through one or more lumens 175 in the body 110. For example, the measuring portion may comprise components optically, acoustically, or electrically connected to proximal components of the measurement assembly such as light or sound emitters or receivers, electrical signal components or electrical power sources. The measurement assembly may be any of the measurement assemblies disclosed in PCT application number PCT/EP2021/078712, which is referenced herein in its entirety.

The indicating device 180 is coupled to the measurement assembly insofar as the difference between the two states of the measurement assembly can be determined (i.e. functionally coupled). The indicating device 180 may be any suitable device which can indicate the difference between the two states. For example, if the measurement assembly provides a qualitative visual difference between the two states, then the indicating device 180 may be a display (e.g. scope display or digital display) configured to display the visual difference to a user of the apparatus, or it may comprise suitable software and/or hardware to receive data (such as image data) from the measurement assembly and perform analysis of the data to determine the state of the measurement assembly (or display the image data). The result of the analysis may be displayed. Alternatively, the measurement assembly may comprise the software/hardware and provide the result of the analysis to the indicating device 180.

The indication provided by the indicating device 180 may be provided to a user of the apparatus 100 if the first actuator 190 is to be actuated manually by the user (for example a visual indication on a display or an audio indication from a speaker), or it may be provided as a command signal to an electronic controller (such as the motion controllers disclosed herein) if the first actuator 190 is to be actuated automatically by the electronic controller. The indication provides a means for the operator or controller to verify that the distal opening is close enough to the endometrial epithelium to deliver the fertilized egg through the inner body.

The first and second actuators 190, 195 may be any suitable actuator controllable to advance the inner body 160 and to expel the fertilized egg. For example, the first actuator 190 may be a pusher or other manual tool for advancing the inner body, or it may comprise a motor system configured to advance the inner body. Likewise, the second actuator 195 may be configured to slide a plunger movable in the inner body to pressurize fluid in the inner body distally and expel the egg. The second actuator may comprise a lead screw for advancing the plunger, i.e. the second actuator may be actuated by applying a torque, and the resulting rotary motion converted into linear motion of the plunger. The rotary actuation of the lead screw allows for a high controllability of the linear motion, and thus the aspirated and dispensed liquid volumes. The first and second actuators 190, 195 may be contained in a housing connected to a proximal end of the inner body 160 and plunger 165.

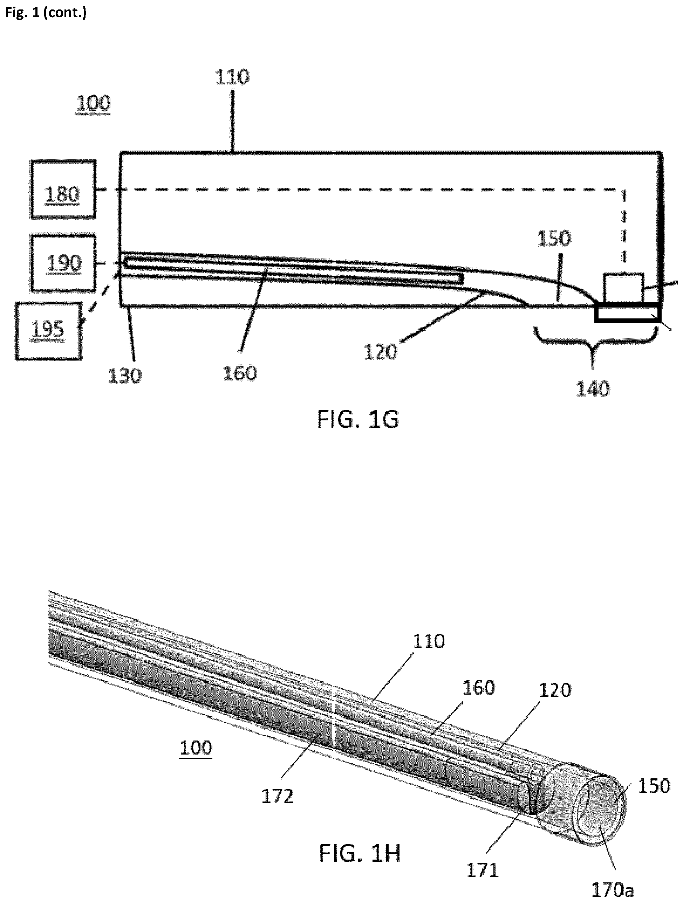

Whilst Fig. 1D shows the measurement portion 170 and distal opening 150 of the body 110 at the distal tip of the body 110, in other embodiments the measurement portion 170 and distal opening of one or more of the lumens such as distal opening 150 may be located at a side of the distal portion 140 of body 110, as shown in Fig. 1G. The lumen 120 comprises a gradually curved section proximal to the distal opening 150 of the body 110 in order to guide the inner body 160 laterally. The covering layer 170a may be provided at least partially on a sidewall of the body 110 to cover one or more distal openings of one or more lumens on a sidewall of the body 110.

Fig. 1C shows the measurement assembly comprising a measurement portion 170 disposed at the distal portion 140 of the body 110. In other embodiments the measurement portion 170 may be located at a different location (e.g. at a distal portion of the inner body 160) or the measurement assembly may be configured to interact with an element of the apparatus 100 in order to provide a qualitative or a quantitative measurement and may comprise any suitable element or elements, without the need of having a measurement portion within the measurement assembly. This is shown in the embodiment of Fig. 1F, wherein the apparatus 100 comprises an inner body 160 having a distal portion 161 comprising a distal end suitable for penetrating the endometrial epithelium. The apparatus 100 further comprises a body 110 (e.g. a catheter) configured to fit within a lumen of the female reproductive system. The body comprises a lumen 120 extending from a proximal end 130 of the body 110 to a distal portion 140 of the body 110 and having a distal opening 150 at the distal portion 140 of the body. The lumen 120 is configured to slidably receive the inner body 160. The apparatus 100 of Fig. 1F further comprises a measurement assembly 162. The measurement assembly 162 is configured to measure whether the apparatus 100 is in a first state indicating the distance between the distal end of the inner body 160 and the endometrial epithelium is greater than a predetermined distance (i.e. in the direction that the inner body is advanced from the distal opening 150 of the body 110), or a second state indicating that the distance between the distal end of the inner body 160 and the endometrial epithelium is equal to or less than a predetermined distance. The apparatus 100 of Fig. 1F further comprises an indicating device 180 coupled to the measurement assembly 162 and configured to indicate that the apparatus 100 is in the first state or the second state. The apparatus 100 also comprises a first actuator 190 operable to advance the inner body 160 out from the distal opening 150 of the body 110, and a second actuator 195 operable to expel a fertilized egg from the inner body (for example to advance a plunger 165).

In an embodiment, the distal portion 161 of the inner body 160 is electrically conductive, and the measurement assembly 162 is configured to make electrical contact with such distal portion 161 of the inner body 160. The measurement assembly thus provides an indication of the capacitance. In an embodiment, the first state is indicated by a capacitance measurement below a threshold value, and the second state is indicated by a capacitance measurement above the threshold value. The covering layer 170a may cover the distal opening 150 to prevent mucus from entering the lumen 120 and affecting the capacitance measurements of the distal portion 161 as the inner body 160 is moved towards the distal opening 150. The inner body 160 is configured to perforate the covering layer 170a so that it can be extended distally from the apparatus 100 to penetrate the endometrial epithelium and transfer the fertilized egg.

In a particular embodiment, the capacitance varies according to the variation of the distance between the distal portion 161 of the inner body 160 and the endometrial epithelium, i.e. the capacitance value changes depending on the proximity of the distal portion 161 of the inner body 160 with the endometrial epithelium. In an embodiment, the predetermined distance is zero (thus meaning contact between the distal portion 161 of the inner body 160 and the endometrial epithelium). In an embodiment, the capacitance value is measured by means of the measurement assembly 162, which comprises an electrical system which is electrically connected to the distal portion 161 of the inner body 160 for measuring the capacitance.

The use of the measurement assembly in combination with the indicating device assists in determining that the distal portion of the apparatus is within a predetermined distance such that the inner body can be advanced into the endometrial epithelium to deliver the fertilized egg. In embodiments wherein the indication provides information regarding the distance between the distal portion of body 110 and the endometrial epithelium, this may assist in avoiding direct contact between the distal portion of body 110 and the endometrial epithelium. Further, prematurely extending the inner body 160 into the uterus whilst the apparatus is still being positioned could cause damage to the inner body 160. Accordingly, the indicating device assists in identifying when it is appropriate to extend the inner body 160 out from the apparatus.

In embodiments wherein the indication provides information regarding the distance between the distal end of the inner body 160 and the endometrial epithelium, the use of the measurement assembly in combination with the indicating device assists in determining that the distal end of the inner body is within a predetermined distance such that the inner body can be further advanced into the endometrial epithelium to deliver the fertilized egg and/or that the inner body is in a position wherein the fertilized egg can be delivered. Accordingly, the indicating device assists in identifying when it is appropriate to deliver the fertilized egg.

In an embodiment, the apparatus 100 of Figs. 1A - 1F further comprises an imaging device, in particular a camera, not shown in the figures (i.e. in addition and separate to the measurement assembly). In an embodiment, the camera is attached to the body 110 and moves together, as an integral device, with said body 110. In a particular embodiment, the camera is housed within the body 110. Fig. 1H shows a perspective view of a distal portion of an apparatus 100 comprising a camera 171 and an inner body 160 slidably received in a single lumen 120. the camera 171 may therefore be removable, and may be sterilised for multiple uses between multiple devices 100. The apparatus of Fig. 1H may comprise all of the features shown in Fig. 1F with the addition of the camera 171 provided in the same lumen 120. There may comprise one or more light sources (not shown) for illuminating an area in front of the camera 171 to be imaged. The one or more light sources may be located proximally to the camera to avoid glare in the image capture by the camera 171. The covering layer 170a covers the distal opening 150 of the lumen 120. The camera 171 may be comprised on an elongate body 172 comprising a longitudinal recess for receiving the inner body 160. The camera 171 may be connected to a display device (not shown) for displaying the field of view of the camera 171 via electrical connections extending through the elongated body 172.

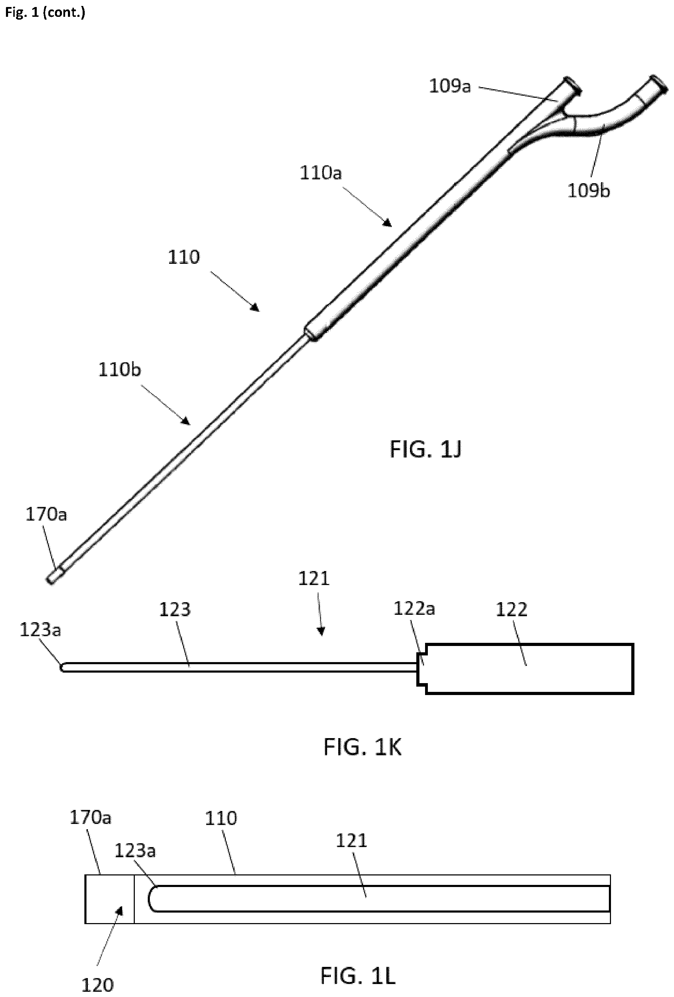

Fig. 1J shows a schematic side view of a body 110 which may be the body 110 as described previously with reference to Figs. 1A to 1H and may form part of apparatus 100 as previously described. The body 110 may comprise a covering layer 170a partially or completely covering a distal end including one or more distal openings of the body 110. A lumen 120 (see Fig. 1L) extends through the body 110 which may be configured to slidably or fixably receive a camera such as camera 171 and an inner body 160 described with reference to Fig. 1H. At a proximal end of the body 110, the body 110 may comprise first and second branches 109a, 109b each having a proximal opening. The lumen 120 branches towards the proximal end of the body 110 to form two lumens, one extending along first branch 109a and another extending along second branch 109a so that the lumen 120 can be accessed via either proximal opening of the first and second branches 109a, 109b. The body 110 may be made of any suitable material and may have any suitable length, particularly the materials and lengths described previously with reference to Figs. 1A to 1H. The body 110 may be a unitary piece or may be formed of several pieces connected by any suitable method such as adhesive or welding. In some embodiments, the body 110 may comprise a distal portion 110b and proximal portion 110a each formed separately and joined together by welding or adhesive to form a single body defining the lumen 120 and branches 109a, 109b.

The lumen of branch 109b may be configured to receive the camera 171 as described with reference to Fig. 1H. In some embodiments, the lumen of branch 109b may comprise a guide such as a protrusion such that the camera 171 may only be inserted into the lumen 120 via branch 109a, so that the elongate body 172 comprising the longitudinal recess is oriented within the lumen 120 at a predetermined orientation. The proximal end of the branch 109b may comprise a connector such as a screw thread for connecting to a corresponding screw thread or Luer lock on a housing at a proximal end of the camera, the housing comprising electrical connections for connecting to electronic cabling for controlling the camera 171 (for example for providing power to the imaging device and one or more light sources of the camera 171 and for receiving image data from the camera 171). The lumen of branch 109a may be configured to receive the inner body 160 as described with reference to Figs. 1A to 1H. The proximal end of branch 109a may comprise a connector for connecting to a housing containing the actuators 190 and 195. For example, the proximal end of branch 109a may comprise a screw thread configured to thread with a corresponding thread on the housing or a Luer lock. Thus, the inner body 160 and plunger 165 may be inserted into the body 110 through branch 109a and fixed to the body 110 by the housing containing the actuators 190 and 195. The inner body 160 and plunger 165 may be advanced and retracted relative to the body 110 by actuating the actuators 190 and 195 as described herein.

Fig. 1K shows a schematic side view of a stylet 121 for an apparatus according to one or more embodiments such as apparatus 100 as described with reference to Figs. 1A to 1J. The stylet comprises a resilient elongate element 123 (e.g. wire or ribbon) configured to slidably extend through body 110. The elongate element 123 is made of a resiliently deformable material which can provide stiffness to the body 110 when it is initially inserted through the cervix during the embryo transfer procedure. For example, the elongate element 123 may be made of a material such as nitinol or stainless steel. The elongate element 123 may have a rounded tip 123a to reduce damage or marring of the inner wall of body 110 defining the lumen 120 when the stylet 121 is inserted into the body 110. In some embodiments, the stylet may comprise a handle 122 to be gripped by the user during insertion into the body 110. The handle 122 may be connected to the elongate element 123 at its distal end, for example by adhesive, welding or a frictional fit. The handle may comprise a connector 122a at its distal end for connecting to a proximal end of the body 110. For example, the connector 122a may comprise a screw thread configured to thread with a screw thread on a proximal end of body 110 (for example proximal end of one of the branches 109a, 109b), or may comprise a Luer lock or other suitable mechanism (such as a frictional fit). The stylet 121 may therefore be fixed to the body 110 during insertion through the cervix and then removed from the body 110.

Fig. 1L shows a schematic cross-sectional side view of the stylet 121 inserted within the lumen 120 of the body 110. The elongate element 123 may have a length which is less than the length of the lumen 120 such than when the stylet 121 is fully inserted into the body 110 and fixedly connected to the body 110 (e.g. by the screw thread, Luer lock or frictional fit), the distal tip 123a of the stylet 121 does not reach the distal end of the body 110. In embodiments where the body 110 comprises a covering layer 170a, this ensures that the covering layer 170a is not pierced by the stylet 121 as the body 110 is advanced through the cervix. For example, in some embodiments, the length of the elongate body 123 may be such that the distal tip 123a is at least 5 mm proximal to the distal end of the body 110 when fully inserted.

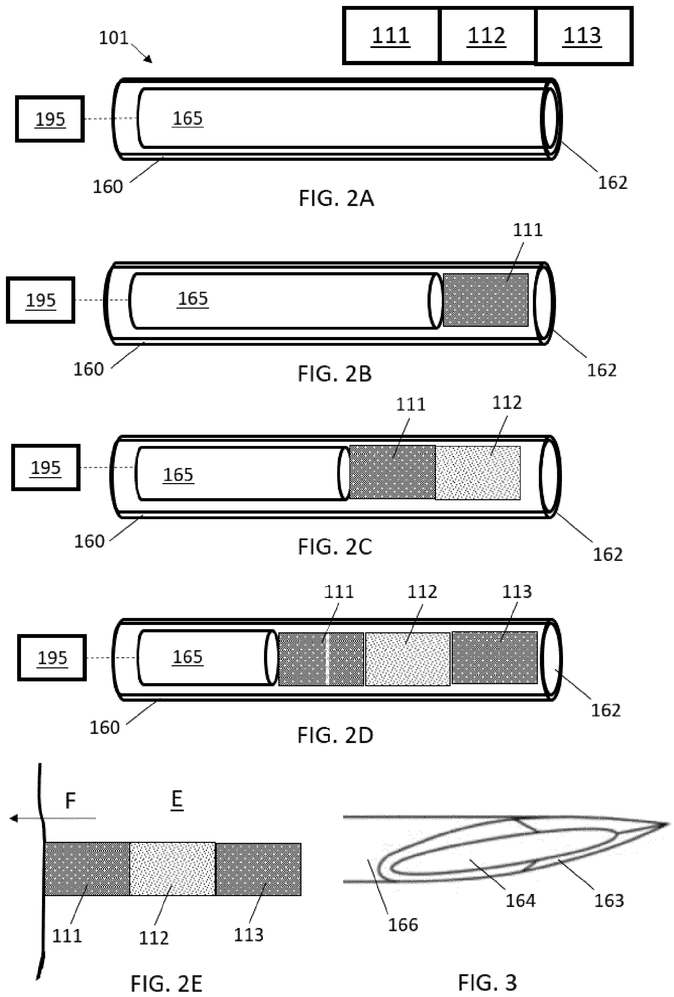

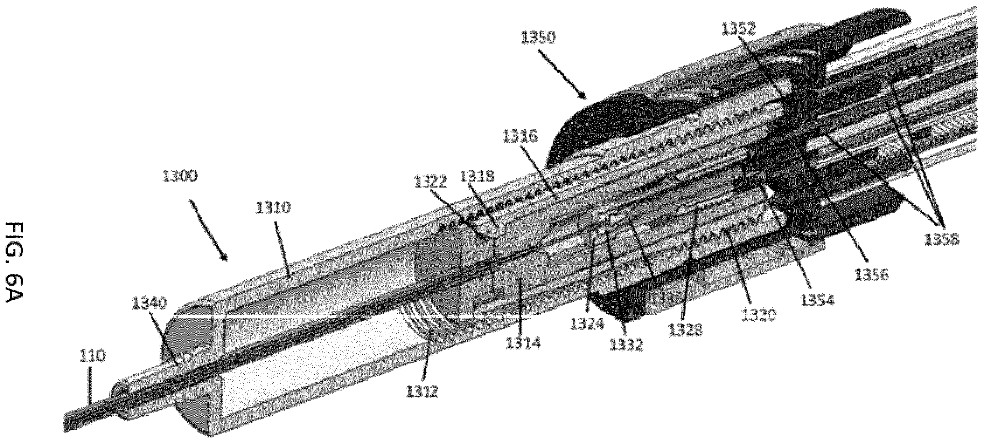

Fig. 2A shows a system 101 for delivering a fertilized egg or embryo into a maternal uterine endometrium in humans or any other mammalian species. The system 101 comprises a body 160 having a distal end 162 suitable for penetrating the endometrial epithelium. The system 101 further comprises a plunger 165 slidably received in a lumen of the body 160, and an actuator 195 operable advance the plunger 165 to expel a fertilized egg from the lumen of the body 160. The actuator 195 may be any suitable actuator for advancing and retracting the plunger 165, and may be a manual actuator such as a proximal end of the plunger 165 which remains accessible to a user when the system 101 is inserted into a lumen of the female reproductive system. The actuator 195 may be any of the actuators disclosed herein any may be a lead screw rotatable to advance and retract the plunger 165 as disclosed with reference to Figs. 6A to 6H. The system 101 further comprises a first injectable medium 111 and a second injectable medium 112 for containing a fertilized egg. The first injectable medium 111 has a higher viscosity than the second injectable medium 112.

The first injectable medium 111 may be any suitable bio-compatible injectable medium that has a higher viscosity than the second injectable medium 112. The second injectable medium 112 may be any injectable medium which is biocompatible and non-toxic to the fertilized egg, as known in the art. It will be understood that the lower bound for the viscosity of the first injectable medium 111 will depend on the viscosity of the second injectable medium 112 selected. In some embodiments, the first injectable medium 111 comprises or consists of one or more of the following: hyaluronic acid, a salt of hyaluronic acid, a biocompatible hydrogel, poloxamer, collagen, fibrinogen, gelatin, sodium hyaluronate and combinations thereof. Use of hyaluronic acid may promote the repair of the endometrial epithelium at the perforation sit of the endometrial epithelium by the body 160.

In some embodiments, there is also provided a third injectable medium 113 also having a higher viscosity than the second injectable medium 112. The third injectable medium 113 may be the same or different viscosity than the first injectable medium 111 and may comprise or consist of the same or different substances to the first injectable medium 111. In some embodiments, the third injectable medium 113 comprises or consists of one or more of the following: hyaluronic acid, a salt of hyaluronic acid, a biocompatible hydrogel, poloxamer, collagen, gelatin, fibrionogen, sodium hyaluronate and combinations thereof.

The first injectable medium 111 and second injectable medium 112 (and optionally the third injectable medium 113) may be provided in medium reservoirs (for example vials or similar) and a user may manually load the system 101 with the injectable mediums by inserting the body 160 into the reservoirs and retracting the plunger 165 to draw in each injectable medium into the lumen of the body 160. In some embodiments, the injectable mediums may be preloaded in the body 160.

In one embodiment, the first injectable medium 111 and the second injectable medium 112 are received (i.e. pre-loaded) in the lumen of the body 160, wherein the second injectable medium 112 is located ahead of the first injectable medium 111 relative to the distal end 162 of the body 160 such that the second injectable medium 112 is expelled before the first injectable medium 111 when the actuator 195 is actuated to expel a fertilized egg. Fig. 2C shows such a configuration. As discussed later in further detail with reference to Fig. 2E, the first injectable medium 111 acts as a plug inhibiting the second injectable medium from being expelled from the endometrial epithelium E.

In one embodiment, the first injectable medium 111, the second injectable medium 112 and the third injectable medium 113 are received (i.e. pre-loaded) in the lumen of the body 160, wherein the second injectable medium 112 is received between the first and third injectable mediums 111, 113, such that the second medium 112 is expelled before of one of the first and third injectable mediums 111, 113, and after the other of the first and third injectable mediums, 111, 113, when the actuator 195 is actuated to expel a fertilized egg. Fig. 2D shows such a configuration. As discussed later in further detail with reference to Fig. 2E, the first injectable medium 111 acts as a plug inhibiting the second injectable medium from being expelled from the endometrial epithelium E and third injectable medium 113 provides additional structural support to maintain the second injectable medium 112 containing the fertilized egg in the desired injected position within the endometrial epithelium E.

The system 101 illustrated in Fig. 2A (and in any of the configurations shown in any of Figs. 2A to 2D) may be provided as shown in Fig. 2A or may be incorporated into any suitable embryo transfer system. For example, the body 160 may be slidably received through a delivery catheter which is positionable within a lumen of the female reproductive system. The system 101 may optionally be incorporated in any of the embryo transfer systems disclosed herein. In particular, the body 160 shown in Figs. 2A to 2D may be the inner body 160 shown in any of Figs. 1B to 1H, and may additionally comprise an body 110 (also referred to as an outer body) configured to fit within a lumen of the female reproductive system, the body 110 comprising an outer lumen 120 extending from a proximal end 130 of the body 110 to a distal portion 140 of the body 110 and having a distal opening 150 at the distal portion 140 of the body 110, the lumen 120 configured to slidably receive the body 160 (also referred to as an inner body). The system may additionally comprise an actuator 190 operable to advance the inner body 160 out from the distal opening 150 of the outer body 110.

A method of loading the system 101 of Fig 2A (whether incorporated into the system described with respect to Figs. 1B to 1H or any other embryo transfer system) may be as follows. In a first step, the first injectable medium 111 may be drawn into the lumen of the body 160. In particular, the body 160 may be inserted into a reservoir containing the first injectable medium 111 and the actuator 195 may be actuated to retract the plunger 165 in order to draw the first injectable medium 111 into the lumen of the body 160 (Fig. 2A to Fig. 2B). In a second step subsequent the first step, the second injectable medium 112 may be drawn into the lumen of the body 160. In particular, the body 160 may be inserted into a reservoir containing the second injectable medium 112 and the actuator 195 may be actuated to retract the plunger 165 in order to draw the second injectable medium 112 into the lumen of the body 160 (Fig. 2B to Fig. 2C). In some embodiments, in a third step subsequent the second step, the third injectable medium 113 may be drawn into the lumen of the body 160. In particular, the body 160 may be inserted into a reservoir containing the third injectable medium 113 and the actuator 195 may be actuated to retract the plunger 165 in order to draw the third injectable medium 113 into the lumen of the body 160 (Fig. 2C to Fig. 2D).

It is noted that the system 101 may be delivered to the endometrial epithelium E in either of the loaded states shown in Figs. 2C and 2D and the actuator 195 may be actuated to deliver the first and second injectable mediums 111 and 112 to the endometrial epithelium E. Fig. 2E shows the injectable mediums 111, 112 and optionally 113 inside the endometrial epithelium E after injection by the system 101. When the system is removed from the endometrial epithelium E after injection, it has been observed that the endometrial epithelium E exerts an expulsive force F on the injected substance and the injectable medium 112 is at risk of being expelled from the endometrial epithelium E immediately after injection, risking a failed fertility treatment procedure. Provision of the first injectable medium 111 in the position shown in Fig. 2B allows the first injectable medium 111 to be the outermost part of the injected substance in the endometrial epithelium E, as shown in Fig. 2E, and provides a high viscosity plug which better resists the expulsive force F and therefore reduces the risk of the endometrial epithelium E expelling the second injectable medium 112 from the endometrial epithelium E. In embodiments where the third injectable medium 113 is used, provision of the third injectable medium 113 in the position shown in Fig. 2D allows the third injectable medium 112 to be the innermost part of the injected substance in the endometrial epithelium E, as shown in Fig. 2E. The first and third injectable mediums 111, 113, therefore provide a pocket within the endometrial epithelium E where the second injectable medium 112 is located, with the first and third injectable mediums 111, 113 providing structural support either side of the pocket to inhibit transmission of the forces, such as the expulsive force F or any inward pressure from the surrounding tissue, to be transmitted to the second injectable medium 112, thereby further increasing the stability of the injected substance when situated in the endometrial epithelium E.

In any of the embodiments disclosed herein with reference to Figs. 1A to 2E, the inner body 160 may comprise a hydrophobic coating formed on a distal portion of the inner body 160. The hydrophobic coating may be formed on one, two or all of an inner surface 164 of the distal portion of inner body 160, the distal tip 163 of the inner body 160 and the outer surface 166 of the distal portion of the inner body 160. Alternatively, or additionally, the inner body 160 may comprise a bevelled tip 163.

When a covering layer is provided covering the distal opening of the lumen containing the inner body 160, the bevelled tip 163 assists in perforating the covering layer. Further, the bevelled tip 163 decreases the force required to penetrate the endometrial epithelium.

The hydrophobic coating may comprise or consist of a polymer or polymer composite, preferably any of the following: parylene, acrylic, polyethylene, polyurethane and polytetrafluorethylene and composites thereof. The hydrophobic coating inhibits sticking of the fertilized egg or embryo to the distal portion of the inner body 160 (for example resulting in the embryo or egg not being expelled in the first place, or being extracted from the endometrial epithelium with the inner body 160) and therefore reduces the risk of a failed egg or embryo transfer procedure.

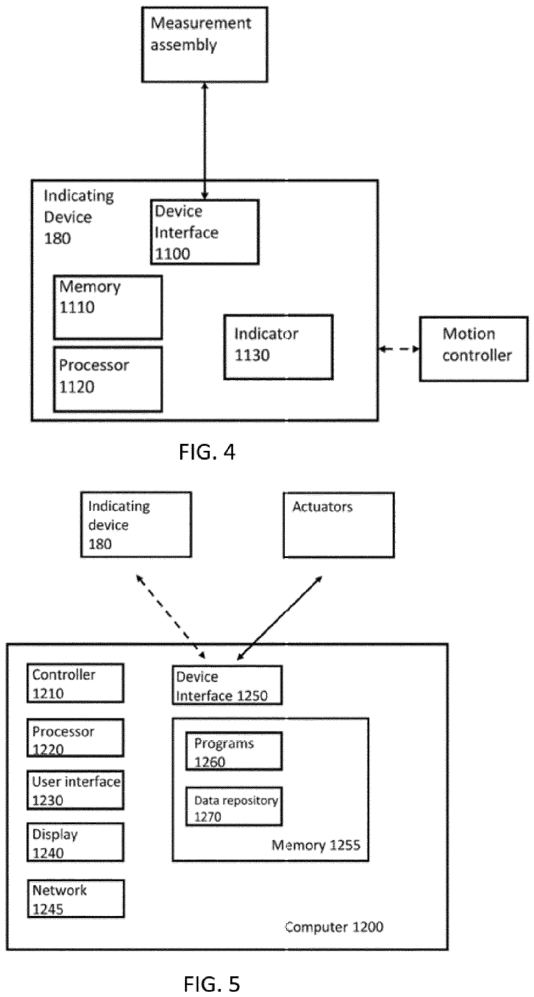

Fig. 4 shows a diagram of an indicating device 180 which may be used for any of the apparatuses disclosed herein comprising a measurement assembly, when the output of the measurement assembly is a signal (for example if the measurement assembly comprises a camera and/or distance measuring probe such as a capacitance sensor). The indicating device comprises a device interface 1100 configured to communicate with (i.e. send and receive signals from) the measurement assembly. The indicating device further comprises a memory 1110, processor 1120 and indicator 1130. It will be appreciated than any suitable computing system may be used for the indicating device 180 and that the device shown in Fig. 4 is one of many devices that could be used. For example, a distributed computer system may be used, for example comprising one or more servers or client computing systems, using any known distributed computing technique. In some examples, a general-purpose computer or any other processing system may be used to implement the methods disclosed herein. Furthermore, the steps disclosed below may be implemented in software, hardware, or any combination of these to achieve the same steps.

It will be appreciated by the skilled person that the indicating device of Fig. 4 may be powered by any suitable powering means. The memory 1110 may comprise one or more volatile or non-volatile memory devices, such as DRAM, SRAM, flash memory, read-only memory, ferroelectric RAM, hard disk drives, floppy disks, magnetic tape, optical discs, or similar. Likewise, the processor 1120 may comprise one or more processing units, such as a microprocessor, GPU, CPU, multi-core processor or similar. The device interface 1100 may comprise wired connections, such as optic, fiber-optic, ethernet or similar, or any suitable wireless communication.

In the case of the measurement assembly making a quantitative measurement (e.g. comprising a distance sensing probe), the memory 1110 may contain suitable instructions for performing a comparison of the received signal to the predetermined distance, and the processor 1120 may be configured to perform the instructions. For example, the indicating device 180 may receive the raw data signal via device interface 1110, the processor 1120 may convert the raw data into a numerical value for the predetermined distance, and the numerical value may be compared to the predetermined distance. If the numerical value is equal to or lower than the predetermined distance, then the processor 1120 may instruct the indicator 1130 to indicate that the predetermined distance has been reached. For example, the indicator 1130 may provide an audio or visual signal to the operator, or may comprise an electronic controller configured to provide a command signal to an electronically controlled motion controller to perform the operation of advancing the needle and expelling the embryo (with the memory 1110 containing suitable instructions for providing control signals to the actuators). The indicating device 180 may also be configured to further indicate to the user when the automatic procedure of advancing the need and expelling the embryo has been completed (for example an audio or visual signal). Alternatively, the indicator 1130 may comprise a display displaying the measured distance alongside the predetermined distance.

In the case of the measurement assembly comprising a camera and the measurement being a qualitative measurement, the indicating device may merely be a display for displaying the real-time image captured by the camera, optionally with a comparison overlay on the display where appropriate. Alternatively, the memory 1110 may store instructions for performing image analysis of the image data to automatically determine if the image indicates that the predetermined distance has been reached. The processor 1120 may be configured to carry out the instructions. The processor 1120 may then instruct the indicator 1130 to indicate that the predetermined distance has been reached. For example, the indicator 1130 may provide an audio or visual signal to the operator, or may provide a command signal to an electronically controlled motion controller to perform the operation of advancing the needle and expelling the embryo.

Whilst Fig. 4 shows the motion controller and the indicating device as separate devices, indicating device and motion controller may be incorporated into a single device. Further, the measurement assembly may instead be connected to the indicating device indirectly via a device interface of the motion controller.

Fig. 5 shows a diagram of a computer 1200 that may be used to control one or more of the actuators disclosed herein. The computer 1200 may comprise one or more of a controller 1210, a processor 1220, a user interface 1230, a display 1240, a network connection 1245, a device interface 1250, and a memory 1255 storing instructions for programs 1260 and a data repository 1270. It will be appreciated than any suitable computing system may be used for the computer 1200 and that the device shown in Fig. 5 is one of many devices that could be used. For example, a distributed computer system may be used, for example comprising one or more servers or client computing systems, using any known distributed computing technique. In some examples, a general-purpose computer or any other processing system may be used to implement the methods disclosed herein. Furthermore, the steps disclosed below may be implemented in software, hardware, or any combination of these to achieve the same steps.

It will be appreciated by the skilled person that the indicating device of Fig. 5 may be powered by any suitable powering means. The memory 1255 may comprise one or more volatile or non-volatile memory devices, such as DRAM, SRAM, flash memory, read-only memory, ferroelectric RAM, hard disk drives, floppy disks, magnetic tape, optical discs, or similar. Likewise, the processor 1220 may comprise one or more processing units, such as a microprocessor, GPU, CPU, multi-core processor or similar. The device interface 1250 may comprise wired connections, such as optic, fiber-optic, ethernet or similar, or any suitable wireless communication.

The device interface 1250 is configured to communicate with (i.e. send and receive control information) one or more electronically controlled actuators. Any of the actuators disclosed herein may be electronically controlled. The device interface 1250 may also be configured to communicate with the indicating device 180. Alternatively, the computer 1200 may comprise all of the features of the indicating device shown in Fig. 4 and may itself be considered as the indicating device 180.

The computer 1200 provides manual control of the electronically controlled actuators by a user of the apparatus. For example, the memory 1255 may comprise instructions for a program 1260 which when executed by processor 1220 causes a graphical user interface (GUI) to be displayed on display 1240, or remotely via network connection 1245. The GUI may comprise appropriate inputs to enable the user to actuate one or more of the actuators. For example, the user may select a function from the GUI, which causes the controller 1210 to send a corresponding control signal to one or more actuators to perform the function.

For example, the user may be able to select a command from the GUI for advancing the body by a certain distance, or at a certain constant speed. The user may be able to select one or more commands for steering the steering mechanism. The user may be able to select one or more commands for advancing the inner body and/or plunger. The commands may correspond to a sequence of commands such that several commands are performed in sequence. For example, the memory 1255 may store instructions for advancing the body 110 until the predetermined distance is reached (i.e. until the indicating device indicates that the predetermined distance is reached), and for subsequently advancing the inner body and plunger to expel the embryo. It may be that all actuators are actuated via such an automated process, or that only some are actuated automatically and the rest are actuated manually (i.e. by user control via the GUI).

The computer 1200 may be configured to receive data from the measurement assembly, either directly or via indicating device 180, and display the data on the GUI alongside the command options. For example, the display 1240 may display real-time image data received from the measurement assembly. The GUI may also display an indication that the predetermined distance has been reached to the user.

In some embodiments, the apparatus may further comprise a motion controller configured to connect to a proximal portion of the body 100, wherein the controller is configured to actuate the first actuator, second actuator, and/or a fourth actuator for advancing the body relative to the motion controller. Any suitable motion controller may be used. For example, the motion controller may comprise one or more linear or rotary mechanisms for actuating the first actuator, the second actuator and/or the fourth actuator. In some embodiments, one or more of the first, second and fourth actuator may comprise a lead screw, each lead screw rotatable to advance the body 110, inner body 160 or plunger 165, the motion controller may be configured to actuate the one or more lead screws. The motion controller may be manually controlled or electronically controlled. The motion controller may be configured to connect to the body via a connector.

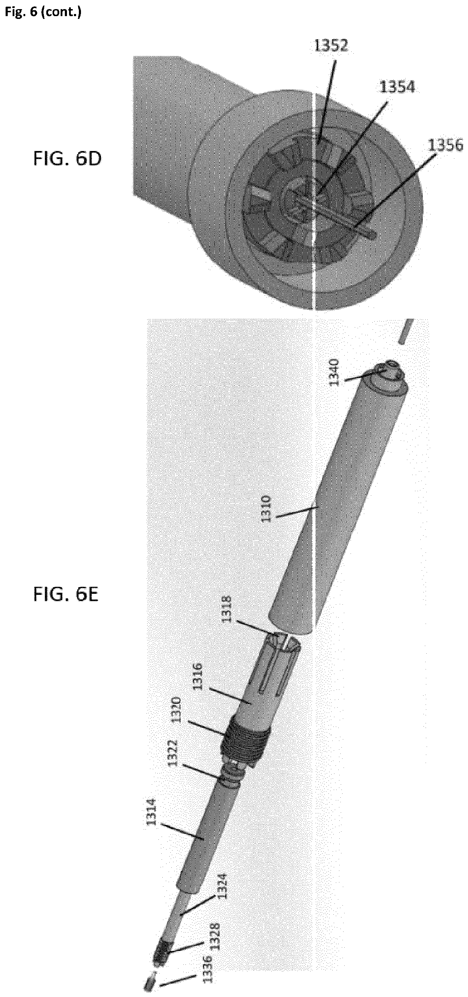

Fig. 6A shows a cross-sectional perspective view of a connector 1300 configured to connect to a motion controller 1350. Fig. 6E shows an exploded view of the connector 1300. The connector 1300 comprises a connector housing 1310 which is insertable into a distal end of the motion controller 1350. The connector 1300 also comprises a body connector 1314 fixedly receiving a proximal portion of the body 110 (e.g. by a frictional fit or by adhesive), an inner body connector 1324 fixedly receiving a proximal portion of the inner body 160 (e.g. by frictional fit or by adhesive) and a plunger connector 1332 fixedly receiving a proximal portion of the plunger 165 (e.g. by frictional fit or by adhesive).

The body connector 1314 is received by the connector housing 1310 and is slidable relative to the connector housing 1310 at a fixed rotational orientation. For example, the connector housing 1310 may comprise a longitudinally extending protrusion or recess and the body connector 1314 may comprise a corresponding recess or protrusion such that the body connector 1314 is only receivable in the connector housing 1310 at a single rotational orientation. The connector 1300 further comprises a body lead screw 1316 having an external screw thread 1320 which corresponds to an internal screw thread 1312 of the connector housing 1310. The body lead screw 1316 abuts the body connector 1314 such that, when turning the body lead screw 1316 to advance the body lead screw distally, the body connector 1314 also advances distally (but in a fixed orientation). In the illustrated embodiment, the body lead screw 1316 is connected to the body connector 1314 by one or more protrusions 1318 which are received by a corresponding recess 1322 in the body connector. The interaction between the protrusion 1318 and the recess 1322 means that the body lead screw 1316 abuts the body connector 1314 when it slides both distally and proximally, meaning the body lead screw 1316 is able to both advance and retract the body connector 1314 and thus the body 110. In some embodiments the protrusion 1318 may instead be located on the body connector 1314 and the recess on the body lead screw 1316.

The inner body connector 1324 is housed by the body connector 1314 and is slidable relative to the connector housing 1310 and the body connector 1314. In some embodiments, the inner body connector 1324 may be slidable relative to the connector housing 1310 and body connector 1314 at a fixed rotational orientation (for example using corresponding protrusions and recessions in the body connector 1314 and the inner body connector 1324 as previously disclosed).

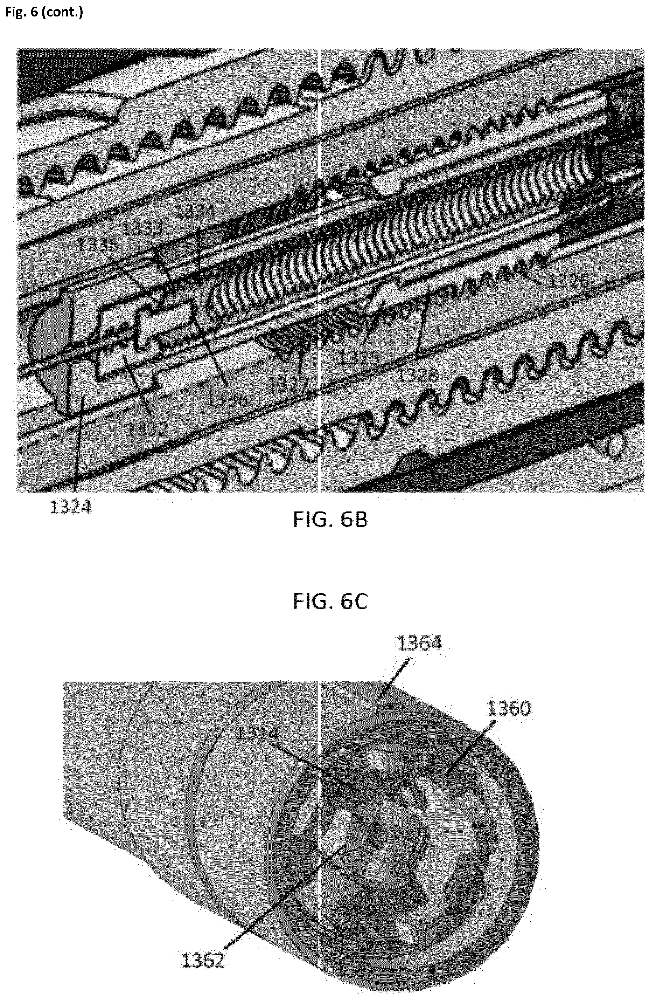

Fig. 6B shows a close up of the inner body connector 1324 and the plunger connector 1332 shown in Fig. 6A. The connector further comprises an inner body lead screw 1328 having an external screw thread 1326 which corresponds to an internal screw thread 1327 of the body connector 1314. The inner body lead screw 1328 abuts the inner body connector 1324 such that, when turning the inner body lead screw 1328 to advance the inner body lead screw 1328 distally, the inner body connector 1324 also advances distally. In the illustrated embodiment, the inner body lead screw 1328 is connected to the inner body connector 1324 by one or more protrusions 1325 which are received by a corresponding recess in the inner body connector 1325. The interaction between the protrusion 1325 and the recess means that the inner body lead screw 1328 abuts the inner body connector 1324 when it slides both distally and proximally, meaning the inner body lead screw 1328 is able to both advance and retract the inner body connector 1324 and thus the inner body 160. In some embodiments the protrusion 1325 may instead be located on the inner body connector 1324 and the recess on the inner body lead screw 1328. In some embodiments, the inner body connector 1324 and inner body lead screw 1328 may be a single unitary body.

The plunger connector 1332 is housed by the inner body connector 1324 and is slidable relative to the connector housing 1310, the body connector 1314 and the inner body connector 1324. In some embodiments, the plunger connector 1332 may be slidable relative to the connector housing 1310, body connector 1314 and inner body connector 1324 at a fixed rotational orientation (for example using corresponding protrusions and recessions in the inner body connector 1324 and the plunger connector 1332 as previously disclosed). The connector further comprises a plunger lead screw 1336 having an external screw thread 1333 which corresponds to an internal screw thread 1334 of the inner body connector 1324. The plunger lead screw 1336 abuts the plunger connector 1332 such that, when turning the plunger lead screw 1336 to advance the plunger lead screw 1336 distally, the plunger connector 1332 also advances distally. In the illustrated embodiment, the plunger lead screw 1336 is connected to the plunger connector 1332 by one or more protrusions 1335 in the plunger connector 1332 which are received by a corresponding recess in the plunger lead screw 1336. The interaction between the protrusion 1335 and the recess means that the plunger lead screw 1336 abuts the plunger connector 1332 when it slides both distally and proximally, meaning the plunger lead screw 1336 is able to both advance and retract the plunger connector 1332 and thus the plunger 165. In some embodiments the protrusion 1335 may instead be located on the plunger lead screw 1336 and the recess on the plunger connector 1332. In some embodiments, the plunger connector 1332 and plunger lead screw 1336 may be a single unitary body.

The connector 1300 may comprises an interlocking feature 1340 at a distal end, such as a Luer lock, for securing the distal end of the connector 1300 to the next distal component of the apparatus.

Fig. 6C shows a perspective view of a proximal end of the connector 1300. Fig. 6D shows a perspective view of a distal end of the motion controller 1350. The proximal end of the connector 1300 is configured to connect with the distal end of the motion controller 1350. The body lead screw 1316 comprises a toothed proximal end 1360 configured to engage with a body lead screw driver 1352 of the motion controller 1350. The inner body lead screw 1328 comprises a toothed proximal end 1362 which is configured to engage with an inner body lead screw driver 1354 of the motion controller 1350. The plunger lead screw comprises a toothed proximal end (not shown) configured to engage with a plunger lead screw driver 1356 of the motion controller 1350. The proximal end of the connector housing 1310 may further comprise a locking feature such as a protrusion 1364 configured to be received by a recess of the distal end of the motion controller (not shown) in order to prevent relative rotation of the distal end of the motion controller 1350 and connector housing 1310. The connector 1300 may be connected to the motion controller 1350 by a push-fit, click-fit, frictional-fit, or similar mechanism. The lead screw drivers 1352, 1354 and 1356 may be distally biased by springs 1358 (see Fig. 6A) in order to ensure engagement with the proximal ends of the lead screws during rotation.

Whilst the illustrated embodiment illustrates a connector which actuates the body connector, inner body connector and plunger connector by the lead screw mechanisms, it will be appreciated that in other embodiments the connector only actuates one or more of the body connector, inner body connector and plunger connector by a lead screw mechanism. For example, the connector may comprise only the body connector 1314, actuated by the body lead screw 1316 as described above, and the needle and plunger may be actuated differently (i.e. by a linear motion mechanism). It will also be appreciated that the outermost screw thread of a given embodiment engages the inner screw thread 1312 of the connector housing 1310. For example, in embodiments where the connector 1300 actuates only the inner body 160 and plunger 165 by the screw thread mechanisms disclosed, the outer thread 1326 of the inner body lead screw 1328 engages with the inner thread 1312 of the connector housing 1310, rather than the inner screw thread of the body connector 1314.

The connector housing 1310, connectors 1314, 1324 and 1332 and lead screws 1316, 1328 and 1336 may be made of any suitable material such as plastic and may be molded or 3D printed. Likewise, the lead screw drivers 1352, 1354 and 1356 may be made of any suitable material such as plastic.

Electrical wiring and/or optical fibers extending from the measurement portion 170 through the body 110 from the distal end of the apparatus may terminate at the connector 1300. In particular, a proximal portion of electrical wiring extending from the distal end of the apparatus through the body 110 may be electrically connected to an electrical terminal of the connector. The motion controller may comprise a corresponding electrical terminal biased towards the electrical terminal of the connector to maintain contact with the electrical terminal of the connector. Similarly, a proximal portion of the optical fibers may be optically connected to an optical terminal of the connector 1300, and the motion controller may comprise an optical terminal configured to form a face seal with the optical terminal of the connector 1300 and biased towards the optical terminal of the connector 1300 to maintain the face seal with the first optical terminal.

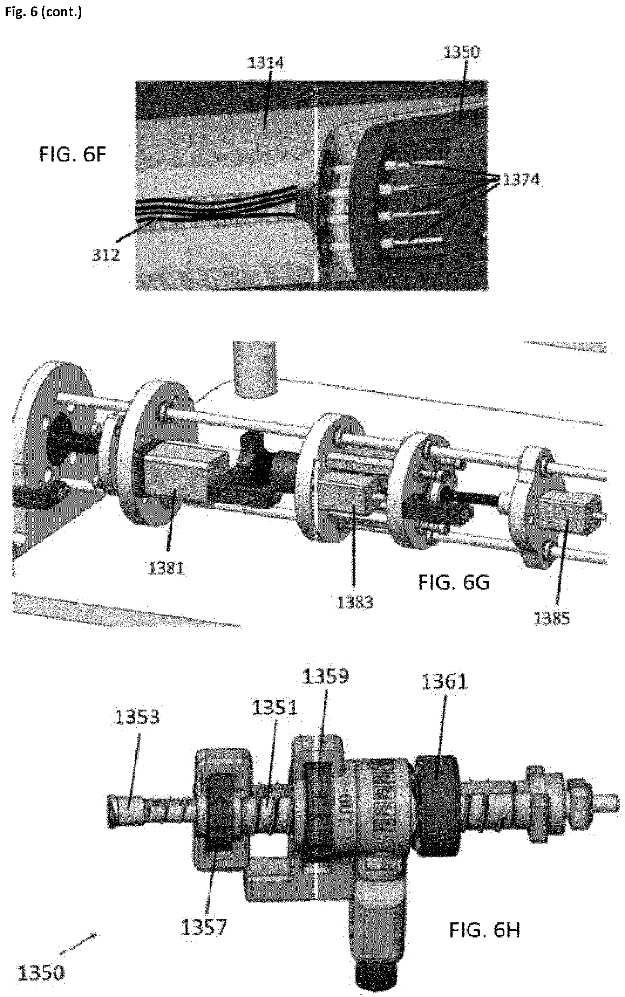

Fig. 6F shows a perspective view of the connection between the body connector 1314 and the distal end of the motion controller 1350. The illustrated embodiment shows a proximal portion of electrical cables 312 which extend from the measurement portion (e.g. camera 171), through the body 110 to the connector 1300. The cables 312 are electrically connected to an electrical terminal (such as a printed circuit board) on the body connector 1314 (e.g. by soldering). The electrical terminal comprises electrical connections which are configured to engage electrical pins 1374 on the motion controller 1350. The electrical pins 1374 are biased distally such that the electrical connection between the pins 1374 and the electrical terminal of the body connector 1314 is maintained as the body connector 1314 is advanced through the connector housing 1300. It will be appreciated that optical connections between the connector 1300 and the motion controller 1350 can be similarly established and maintained.

Fig. 6G shows a perspective view of a proximal portion of the motion controller 1350. The motion controller 1350 comprises a first motor 1381, a second motor 1383 and a third motor 1385. In one embodiment, the first motor 1381 may be configured to drive the body lead screw driver 1352, the second motor 1383 may be configured to drive the inner body lead screw driver 1354 and the third motor may be configured to drive the plunger lead screw driver 1356. The motion controller 1350 may be configured to advance/retract the body 110, inner body 160 and plunger 165 at the same rate by running the first, second and third motors 1381, 1383, 1385 in unison at the rate corresponding to the same advancement speed of the body 110, inner body 160 and plunger 165. The motion controller 1350 may also be configured to advance/retract the inner body 160 and plunger 165 at the same rate relative to the body 110 by running the second and third motors 1383, 1385 in unison at the rate corresponding to the same advancement speed of the inner body 160 and plunger 165. The motion controller 1350 may also be configured to advance/retract the plunger 165 relative to the inner body 160 by running the third motor 1385 alone.

Alternatively, the first motor 1381 may be configured to turn each of the lead screw drivers 1352, 1354 and 1356 (e.g. via a first threaded rod) to advance the lead screws 1316, 1328 and 1336 at the same rate (to advance the body 110, the inner body 160 and the plunger 165 at the same rate). The second motor 1383 may be configured to turn the inner body lead screw driver 1354 and the plunger lead screw driver 1356 (e.g. via a second threaded rod) to advance the inner body lead screw 1328 and the plunger lead screw 1336 at the same rate (to advance the inner body 160 and plunger 165 at the same rate). The third motor 1386 may be configured to turn the plunger lead screw driver 1356 (e.g. via a threaded rod) to advance the plunger 165.

The motion controller 1350 may further comprise an axial motor (not shown) to retract the plunger lead screw driver 1352 before connection or disconnection of the connector 1300, in order to prevent damage to the plunger lead screw driver 1352 during connection or disconnection of the connector 1300.

The connector 1300 may be assembled in the following steps (the steps need not necessarily be performed in chronologically in the order presented). The proximal portion of the plunger 165 may be fixed to the plunger connector 1332. The plunger connector 1332 may be connected to the plunger lead screw 1336 (for example the connector and lead screw are pushed together until the protrusion falls into the recess to secure the connector and lead screw together). The proximal portion of the inner body 160 may be connected to the inner body connector 1324. The inner body connector 1324 may be connected to the inner body lead screw 1328 (e.g. by pushing the connector and lead screw are together until the protrusion falls into the recess to secure the connector and lead screw together). The proximal portion of the body 110 may be connected to the body connector 1314. The body connector 1314 may be connected to the body lead screw 1316 (e,g. by pushing the connector and lead screw are together until the protrusion falls into the recess to secure the connector and lead screw together). The distal end of the plunger 165 may be passed through the inner body connector 1324 from the proximal end and through the inner body 160. The plunger lead screw 1336 may be screwed to the inner body connector 1324. The distal end of the inner body 160 may be passed through the body connector 1314 from the proximal end and through the body 110. The inner body lead screw 1328 may be screwed to the body connector 1314. The distal end of the body may be passed through the connector housing 1310 from the proximal end. The body lead screw 1316 may be screwed to the connector housing 1310. The lead screws may then be actuated to moved the body 110, inner body 160 and plunger 165 to the correct relative positions.

When the connector 1300 is assembled, the plunger lead screw 1336 may be actuated to aspirate the fluid containing the embryo. The connector 1300 is then connected to the motion controller 1350.

The length from the proximal end of the body connector 1314 to the distal tip of the body 110 may be about 300mm to 400mm, preferably 325mm to 375mm, for example 358mm. The maximum length of the connector 1300 from the proximal end to the distal tip of the body 110 may be bout 400mm to 500mm, preferably 425mm to 475mm, for example 450mm.

Fig. 6H shows a perspective view of a motion controller 1350 according to an embodiment. The motion controller 1350 is configured to connect to a proximal portion of the body 110 and to actuate on said body 110. In the embodiment shown in Fig. 6H, the motion controller 1350 comprises two lead screws 1351, 1353 configured to advance/retract the body 110, and to rotate and advance/retract an outer catheter which houses the body 110 in an embodiment. In the embodiment shown the motion controller 1350 further comprises three actuation mechanisms 1357, 1359, 1361, each actuation mechanism configured to actuate on one of the lead screws 1351, 1353. The actuation of the lead screws 1351, 1353 may be manual or may be motorized. Although in this embodiment two lead screws 1351, 1353 are shown, in other embodiments there may be only one lead screw, depending on the elements intended to be moved by the motion controller 1350. The motion controller 1350 allows fine and precise control of the movement of the body 110 and of the outer catheter housing the body, as well as fixing the outer catheter in the required position. In an embodiment, the motion controller 1350 may be actuated based on a real time image obtained by an imaging device, such as a camera, present in the apparatus 100.

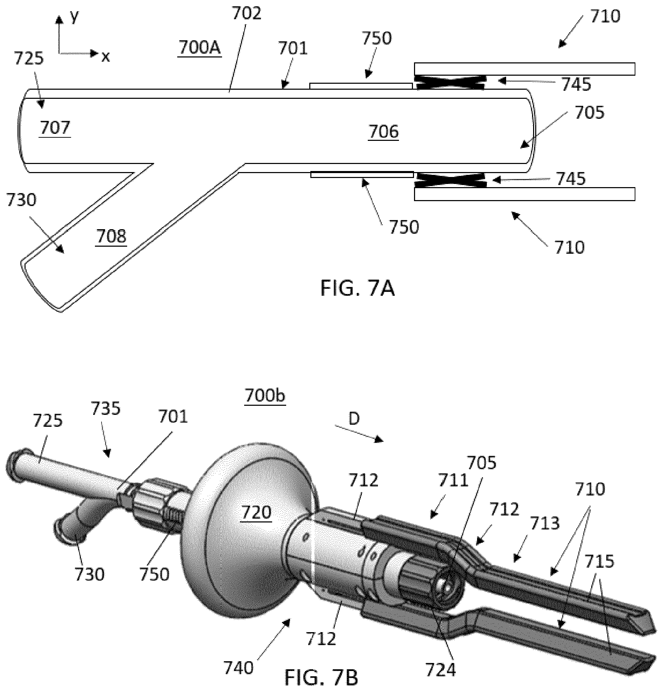

Fig. 7A shows a cross-sectional side view of a device 700a for engaging a speculum (also referred to as a speculum lock). The device 700a comprises two or more expandable engaging elements 710 for outwardly engaging a speculum to fix the device to a speculum, wherein the two or more expandable engaging elements 710 comprise a plurality of expanded configurations for engaging a plurality of speculum sizes. The device 700a comprises a guide 705 for guiding an embryo delivery device between the engaging element 710 and through a speculum when the device 700a is fixed to the speculum. In particular, the guide 705 at least partially conforms to an outer surface of the embryo delivery device to allow longitudinal movement of the embryo delivery device through the guide (i.e. in the x-direction) and to prevent lateral movement of the embryo delivery device in the guide (i.e. in the y-direction). The guide 705 may comprise a tubular body 701 having a tubular wall 702 defining a lumen 705 for slidably receiving the embryo delivery device, wherein the cross-sectional shape of the inner surface of the tubular wall 702 at least partially conforms to the outer surface of the embryo delivery device to allow longitudinal movement of the embryo delivery device through the guide and to prevent lateral movement of the embryo delivery device in the guide. The embryo delivery device may be the body 110 incorporated in any of the apparatuses for delivering a fertilized egg described with reference to Figs. 1A to 6H.

The expandable engaging elements 710 may be coupled to the tubular body 701. The engaging elements 710 may be expandable using any suitable mechanism. For example, the device 700a may comprise one or more biasing elements such as a spring connected between the tubular body 701 and the engaging elements 710 to bias the engaging elements 710 to an expanded configurations. The engaging elements 710 may be maintained in a collapsed configuration by a sheath or cover over the engaging elements 710. The engaging elements 710 may be inserted into the speculum and the sheath or cover may be removed (e.g. retracted) from the engaging elements 710 so that the engaging elements 710 are biased to the expanded configuration to engage opposing sides of the speculum to fix the device 700a to the speculum. Other mechanisms may be used, such as a motor connected to the engaging elements 710 for moving the engaging elements between a collapsed configuration and one or more expanded configurations. In some embodiments, the engaging elements 710 may be mounted to the guide 705 via respective scissor lifts 745. The scissor lifts 745 may comprise one or more pairs of struts pivotally connected to one another, with one strut being pivotally mounted to the guide 705 (for example tubular body 701) and the respective engaging element 710, and the other being slidably mounted to the guide 705 (for example tubular body 701) and the respective engaging element. Compression of the scissor lift 745 in a longitudinal direction translates into movement of the engaging elements 710 in a lateral direction, to move to the expanded configurations. The device 100 may comprise one ratcheting actuators 746 slidably mounted to the guide 705 and configured to move the scissor lifts 745 between a plurality of positions corresponding to a plurality of expanded configurations of the expandable engaging elements 710. The ratcheting actuator 746 comprises a plurality of teeth (not shown) engaging with respective teeth on the guide 705 to allow movement in the distal direction D but not in the opposite direction. The device 700a may further comprise a release mechanism (not shown) for releasing the ratcheting mechanism (i.e. by disengaging the teeth) and allowing movement in the proximal direction opposite the distal direction D and therefore movement from the expanded configuration to the collapsed configuration.

In some embodiments, the guide 705 may comprise first branched guide 725 and second branched guide 730 at its proximal end defining respective lumens 707, 708 which branch from lumen 706. The branched guides 725 and 730 allow for separable components of the embryo transfer device (for example the measurement assembly and the inner body) to be inserted through the device 700a or 700b separately, whilst moving in unison when the device 700a or 700b is moved to position the embryo transfer device within the uterus.

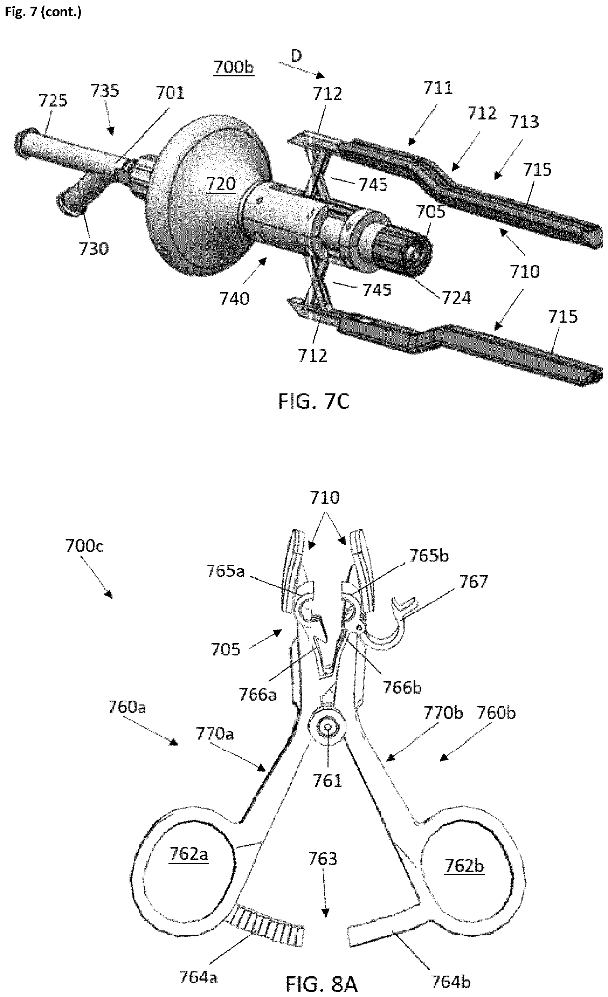

The expandable engaging elements 710 of Fig. 7A may be rectilinear as illustrated or may comprise any of the features of the engaging elements 710 disclosed with reference to Figs. 7B and 7C.

Fig. 7B shows a perspective view of another device 700b for engaging a speculum (also referred to as a speculum lock) in a collapsed configuration. Fig. 7C shows the device 700b of Fig. 7B in an expanded configuration. Again, the device 700b comprises two or more expandable engaging elements 710 for outwardly engaging a speculum to fix the device 700b to the speculum, wherein the two or more expandable engaging elements 710 comprise a plurality of expanded configurations for engaging a plurality of speculum sizes. The device further comprises a guide 705 for guiding an embryo delivery device between the expandable engaging elements 710 and through the speculum when the device 700b is fixed to the speculum. The guide 705 may comprise a tubular body 701 having a tubular wall defining a lumen for slidably receiving the embryo delivery device, wherein the cross-sectional shape of the inner surface of the tubular wall 702 at least partially conforms to the outer surface of the embryo delivery device to allow longitudinal movement of the embryo delivery device through the guide and to prevent lateral movement of the embryo delivery device in the guide.

The engaging elements 710 may be expandable using any suitable mechanism as described with reference to Fig. 7A. **In** the illustrated embodiment, the engaging elements 710 may be mounted to the guide 705 via respective scissor lifts 745. The scissor lifts 745 may comprise one or more pairs of struts pivotally connected to one another, with one strut being pivotally mounted to the guide 705 (for example tubular body 701) and the respective engaging element 710, and the other being slidably mounted to the guide 705 (for example tubular body 701) and the respective engaging element. The device 700b comprises a proximal part 735 and a distal part 740, which are slidably connected. In the illustrated embodiments, the proximal part 735 is slidably received within a lumen of the distal part 740. One strut of each scissor lift 745 is pivotally mounted to the proximal part 735 whilst the other is pivotally mounted to the distal part 740. When the proximal and distal parts 735, 740 are moved relative to one another, the scissor lifts are actuated so that the expandable engaging elements move between various expanded configurations. The device 700b also comprises a ratcheting actuator 750 comprising a plurality of teeth on the proximal part 735 engaging with respective teeth on the distal part 740 to allow relative movement of the proximal part 735 relative to the distal part 740 in one direction but not in the opposite direction. The device 700a may further comprise a release mechanism (not shown) for releasing the ratcheting mechanism (i.e. by disengaging the teeth) and allowing movement in the opposite of the proximal part 735 relative to the distal part 740 and therefore movement from the expanded configuration to the collapsed configuration. It will be appreciated that the direction of relative movement of the proximal part 735 and the distal part 740 which causes the engaging elements 710 to move to the expanded position will depend on which strut is pivotally attached to which part. In the illustrated embodiment, the struts are attached such that movement of the proximal part 735 in the distal direction D causes the engaging elements to move outwardly to move to the expanded configurations.

In some embodiments, one or more of the expandable engaging elements 710 comprise a strut 712 movable between a collapsed configuration (as shown in Fig. 7A) and a plurality of expanded configurations (as shown in Fig. 7B). The strut 712 may be rectilinear as illustrated in Fig. 7A or may have an outer portion 711, an inner portion 713 having a lower radial extent than the outer portion 711, and an intermediate portion 712 connecting the outer portion 711 and the inner portion 713. The struts of the scissor lift 745 are pivotally/slidably mounted to an inside surface of the outer portion 711. In any of the embodiments, the expandable engaging elements 710 may further comprise a frictional layer 715 mounted on the strut 712 and made of a material having a higher friction coefficient than the strut 712. The strut 712 provides structural support for the engaging element whilst the frictional layer provides a contact region for the speculum. The strut 712 may be made of for example, a metal and the frictional layer 715 may be made of any suitable material having a higher friction. The use of an outer portion 711 and an inner portion 713 allows the component parts of the device 700b external to the speculum to be less compact whilst still allowing a low radial profile at the distal end of the device 700b ready for insertion into the speculum, as can be seen in Fig. 7B.

The device 700b may comprise one or more branches at a proximal end. In the illustrated embodiment, the device 700b includes first and second branched guides 725 and 730 at its proximal end defining respective lumens which branch from the lumen of the tubular body 701.

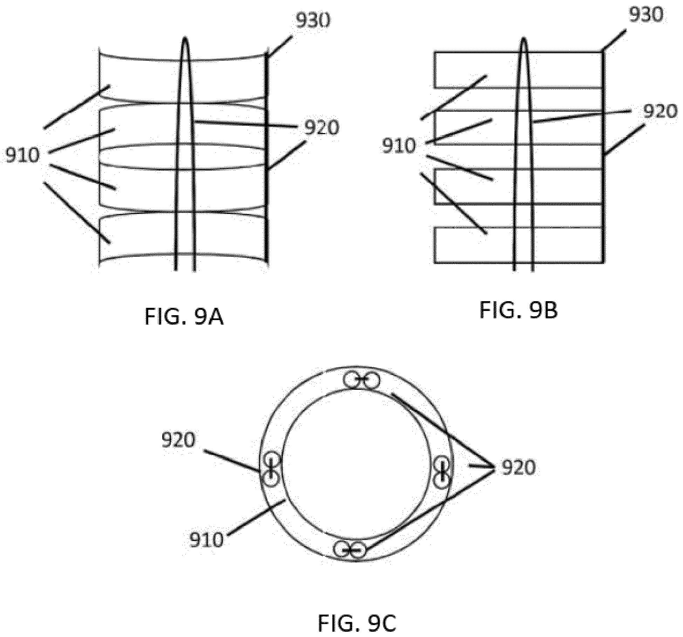

The device 700b may also comprise a connecting mechanism 724 at the distal end of the guide 705, such as a Luer lock or a stab-fit connection, for connecting to a proximal end of another component of an embryo delivery apparatus such as the steering mechanism disclosed in Figs. 9A to 9H. The device 700b may further comprise a handle 720 fixed to the distal part 740.

In use, a speculum may be inserted into the vagina to dilate it. An embryo delivery device may be passed through the device 700a or 700b, through the speculum and past the cervix. The engaging elements 710 may be expanded to the expanded configuration so that the device 700a or 700b is fixed to the speculum. As a result, the embryo delivery device has restricted movement relative to the anatomy of the patient, allowing a higher controllability of the embryo delivery device.

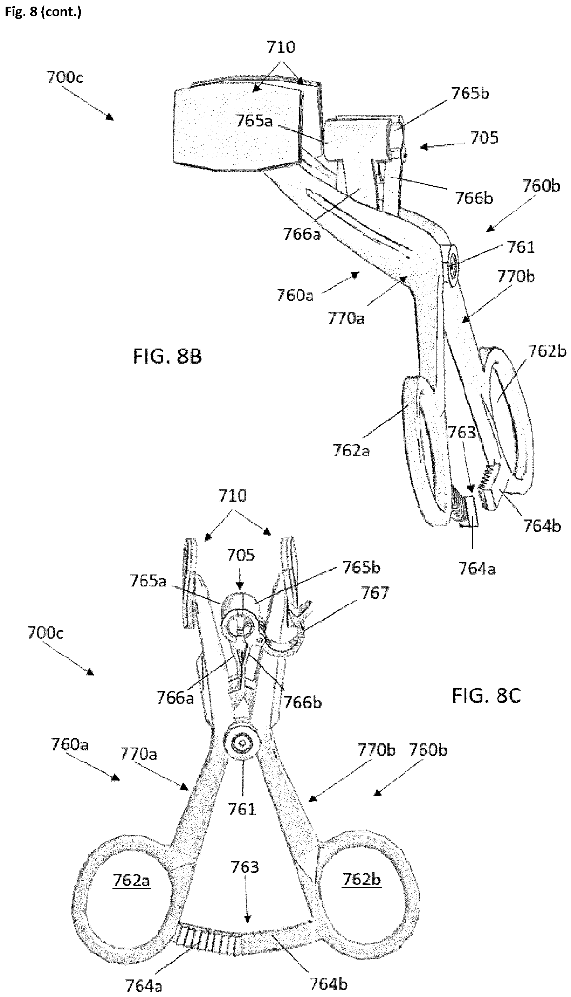

Fig. 8A shows a device 700c for engaging a speculum. The device 700c comprises two or more expandable engaging elements 710 for outwardly engaging a speculum to fix the device to a speculum, wherein the two or more expandable engaging elements 710 comprise a plurality of expanded configurations for engaging a plurality of speculum sizes. The device 700c comprises a guide 705 for guiding an embryo delivery device between the engaging elements 710 and through a speculum when the device 700c is fixed to the speculum. In particular, the guide 705 has a configuration in which it at least partially conforms to an outer surface of the embryo delivery device to allow longitudinal movement of the embryo delivery device through the guide (i.e. in the x-direction) and to prevent lateral movement of the embryo delivery device in the guide (i.e. in the y-direction). In some embodiments, guide 705 has an open configuration in which the embryo delivery device is released, as shown in Fig. 8A. Fig. 8B shows a side view of the device 700c in the same configuration as shown in Fig. 8A.

The device 700c comprises first 760a and second 760b halves pivotably connected by a pivot joint 761 about which the halves 760a and 760b articulate. Each half 760a, 760 comprises a handle 762a, 762b and an expandable engaging element 710 for outwardly engaging a speculum, wherein each respective handle 762a, 762b is connected to the respective expandable engaging element 710 via a lever 770a, 770b. In the illustrated embodiment, the expandable engaging elements 710 are connected to the handles 762a, 762b and the pivot joint 761 such that movement of the handles 762a, 762b together causes the expandable engaging elements 710 to move apart, and movement of the handles 762a, 762b apart causes the expandable engaging elements 710 to move together. In other embodiments, the expandable engaging elements 710 may be connected to the handles 762a, 762b in a scissor arrangement such that movement of the handles 762a, 762b together causes the expandable engaging elements 710 to move together and movement of the handles 762a, 762b apart causes the expandable engaging elements 710 to move apart. Whilst the handles 762a, 762b are illustrated as loops, the handles may take any suitable shape without departing from the scope of the disclosure.

The device 700c may comprise a ratcheting mechanism 763 configured to allow the handles 762a, 762b to move in the direction which causes the engaging elements 710 to move apart and inhibiting the handles 762a, 762b to move in the opposite direction. In the illustrated embodiment, the ratcheting mechanism 763 comprises a set of teeth 764a, 764b on each half 760a, 760b configured to engage with each other such that movement of the handles 762a, 762b together engages the sets of teeth and inhibits movement apart of the handles 762a, 762b. The teeth 764a, 764b may be disengaged by displacing the handles 762a, 762b in a distal and proximal direction respectively (i.e. in a direction perpendicular to the teeth 764a, 764b) to allow the handles 762a, 762b to move apart.

In the embodiment of Fig. 8A, the guide 705 comprises opposing side walls 765a, 765b each shaped to at least partially conforms to an outer surface of the embryo delivery device. side wall 765a is connected to a first of the halves 760b via a flexible arm 766a and side wall 765b is connected to the other of the halves 760a via a flexible arm 766b. The side walls 765a are connected to the halves 760a, 760b such that the side walls 766a and 766b move towards each other when the engaging elements 710 move apart from each other. The flexible arms 766a, 766b may be made of any suitable resiliently deformable material such that the flexible arms 766a, 766b are configured to flex when the side walls 765a, 765b abut one another. This allows the expandable engaging elements 710 to move over a range of positions even when the side walls 765a, 765b abut one another, allowing a wider range of speculum sizes to be engaged by the device 700c.

The device 700c may further comprise a guide locking mechanism 767 having a first configuration in which the guide locking mechanism 767 disengages the guide 705, and a second configuration in which the guide locking mechanism 767 engages and grips the guide 705. In the illustrated embodiment, the guide locking mechanism 767 comprises a grip pivotably connected to one of the side walls 765a, 765b. The grip is movable to a closed configuration in which it covers both side walls 765a, 765b and exerts a compressive force on the guide 705 such that the embryo delivery device is tightly gripped. The guide 705 may be provided proximal of the expandable engaging elements 710 (see Fig. 8B) so that movement of the grip is not obstructed by the expandable engaging elements 710. Whilst in the illustrated embodiment the guide locking mechanism 767 is a pivotable grip, in other embodiments the guide locking mechanism 767 may be any suitable mechanism which is actuatable to provide a compressive force on the guide 705, for example a clip, clamp or a screw mechanism configured to compress the guide 705.

The device 700c may be generally made of any suitable material such as a resilient plastic (for example thermoplastic polymers, e.g. polycarbonate, or similar) or metal (e.g. stainless steel, brass) or combinations thereof. The engaging elements 710 may comprise a frictional coating having a higher friction coefficient than the material of the device 700c, such as silicone rubber, to increase the frictional force exerted by the engaging elements 710 onto the speculum. Similarly, the inner wall of the guide 705 (i.e. the radially inward surfaces of the side walls 766a, 766b) may comprise a frictional coating having a higher friction coefficient than the material of the device 700c, such as silicone rubber, to increase the friction between the embryo delivery device and the guide 705. In the case of deformable materials like silicone rubber, the coating may additionally provide flexibility in adapting to the particular shape of the speculum and embryo transfer device.

Fig. 8C shows the device 700c in a second configuration in which the expandable engaging elements 710 are moved to an expanded configuration to engage a speculum. The side walls 765a, 765b are moved to a closed configuration forming a lumen for engaging the embryo delivery device. The guide locking mechanism 767 is in a disengaged configuration so that sliding movement of the embryo delivery device in the proximal or distal direction (i.e. towards or away from the endometrial wall) is permitted.

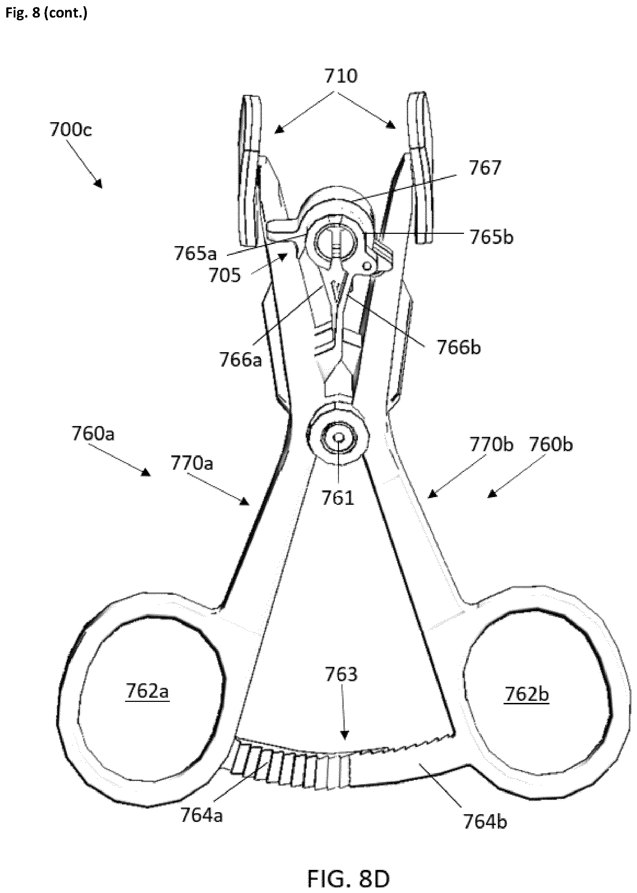

Fig. 8D shows the device 700c in a third configuration in which the expandable engagement elements 710 remain in the expanded configuration to engage the speculum, and further in which the guide locking mechanism 767 engages the guide 705 to provide a compressive force to the guide 705. In this configuration, sliding movement of the embryo delivery device is inhibited by the compressive force exerted by the guide locking mechanism 767.

In use, a speculum may be inserted into the vagina to dilate it. The device 700c in the first configuration of Fig. 8A may be placed such that the expandable engaging elements 710 are inserted into the speculum and the body 110 (such as that shown in Fig. 1J) is positioned approximately within the side walls 765a, 765b. The device 700c may then be moved to the second configuration shown in Fig. 8C. In the second configuration, the expandable engaging elements 710 engage the inside of the speculum such that the device 700c is fixed to the speculum. Further, the side walls 765a, 765b abut one another to form a lumen for containing and conforming to body 110, thus forming guide 705 in which lateral movement of the body 110 is inhibited but sliding movement of the body 110 is permitted. Next, a device such as body 110, optionally with a stylet 121 inserted, may be inserted through the guide 705 and through the cervix to a preliminary position.

It is noted that in some embodiments, body 110 (with or without a stylet 121) may first be inserted through the vagina, through the cervix and into the uterus after the speculum is inserted through the vagina but before the device 700c is inserted into the speculum. After successful insertion of the body 110, device 700c may be inserted into the speculum in the first configuration shown in Fig. 8A. The device 700c may then be moved to the second configuration shown in Fig. 8C with the body 100 being held between the side walls 765a, 765b until the device 700c engages the speculum and the body 100.

Once the device 700a is locked to the speculum and the body 110 is correctly positioned in the uterus, the guide locking mechanism 767 may then be moved to a closed position to inhibit further sliding movement of the body 110. The stylet 121 may be removed from the body 110. A camera 171 may be inserted into the body 110 and connected to a display device and power supply such that a real-time image of the distal end of the body 110 may be viewed by the user. If necessary, the guide locking mechanism 767 may be released to allow the insertion depth of the body 110 to be adjusted until it is determined that the distal end of the body 110 is close to the fundus, after which the guide locking mechanism 767 is closed again to inhibit sliding movement of the body 110. An inner body 160 with a fertilized egg or embryo loaded may then be inserted into body 110 and actuator 190 may advance the inner body 160 to the endometrial epithelium. Once it is determined that the inner body 160 has reached the endometrial epithelium (for example by observing a change in the capacitance measured by a capacitance sensor electrically connected to inner body 160), the inner body 160 is advanced a predetermined distance into the endometrial epithelium and the plunger is advanced to deploy the fertilized egg or embryo.

It is noted that the devices 700a, 700b and 700c may be used in conjunction with any suitable tertiary device for insertion into the body through a speculum, in addition to embryo transfer devices.

Fig. 9A shows a side view of a steering mechanism configured to steer a distal portion of the body 110 according to some embodiments. The steering mechanism comprises a plurality of connected rings 910 forming a tube wall configured to at least partially surround a portion of the body 110. Specifically, the rings 910 are sized to receive the body 110 inside the tube wall, and may slidably receive the body 110. The mechanism further comprises at least one cable 920 extending longitudinally along the tube wall and attached to a distal end 930 of the tube wall. For example, the at least one cable may extend longitudinal along the inner or outer side of the tube wall or may extend longitudinally through the tube wall. The mechanism may comprise a plurality of said cables located at different circumferential positions about the tube wall. The connected rings 910 may be connected by the cables 920 alone. The connected rings 910 may further be configured to maintain a predetermined alignment by comprising a set of interlocking features which inhibit the rings 910 from rotating about the longitudinal axis. The steering mechanism shown in Fig. 9A comprises a plurality of connected rings 910 which are pivotable about each other. In other words, the connected rings are undulated such that neighbouring rings abut at a tangential contact point. This enables the rings 910 to roll about each other to steer the body 110 when one of the cables 920 are retracted by the third actuator (not shown). The circumferential position of the tangential contact points about which the ring 910 roll may be varied along the length of the steering mechanism. For example, sequential circumferential contact points may be offset by 90 degrees in order to accommodate rolling in two perpendicular directions by a plurality of cables. Preferably, the steering mechanism comprises two pairs of said cables, each pair comprising radially opposing cables, in order to provide steering in a plurality of directions. Such a configuration is shown in Fig. 9C, which is a top view of such a steering mechanism.

The connected rings shown in Fig. 9A may be made of any suitable material such as medical grade injection moldable or 3D printed plastic resin such as ABS, PET, DELRIN or PTFE. The cables 920 may be made of, for example, stainless steel and threaded through holes formed in the walls of the connected rings forming the tube wall. The cables 920 may each extend through a pair of holes in the connected rings and be looped at the distal end 930 as shown in Figs. 9A and 9C. The third actuator may be any suitable actuator for contracting the one or more cables.

Fig. 9B shows a side view of a steering mechanism according to an alternative embodiment. The steering mechanism comprises a plurality of connected rings 915 forming a tube wall configured to at least partially surround a portion of the body 110. Specifically, the rings 915 are sized to receive the body 110 inside the tube wall, and may slidably receive the body 110. The mechanism further comprises at least one cable 920 extending longitudinally through the tube wall and attached to a distal end 930 of the tube wall. The plurality of connected rings 910 are rings of a compressible tube or spring. When one of the cables is retracted by the third actuator (not shown), the tube or spring is compressed on the side of the retracted cable such that the steering mechanism is steered towards the contracted side. As the tube is compressible, the tension force is partly counteracted such that the resulting curve of the mechanism shown in 9B may be less than for the embodiment shown in Fig. 9A for a given tensioning force. Once again, preferably, the steering mechanism comprises two pairs of said cables, each pair comprising radially opposing cables, in order to provide steering in a plurality of directions, as shown in Fig. 9C.

The connected rings shown in Fig. 9B may be a compressible tube or spring made of any suitable material such as FEP, PTFE. The cables may be made of any suitable material such as stainless steel and threaded through holes formed in the walls of the connected rings forming the tube wall. The cables 920 may each extend through a pair of holes in the connected rings and be looped at the distal end 930 as shown in Figs. 9A and 9C. The third actuator may be any suitable actuator for contracting the one or more cables.

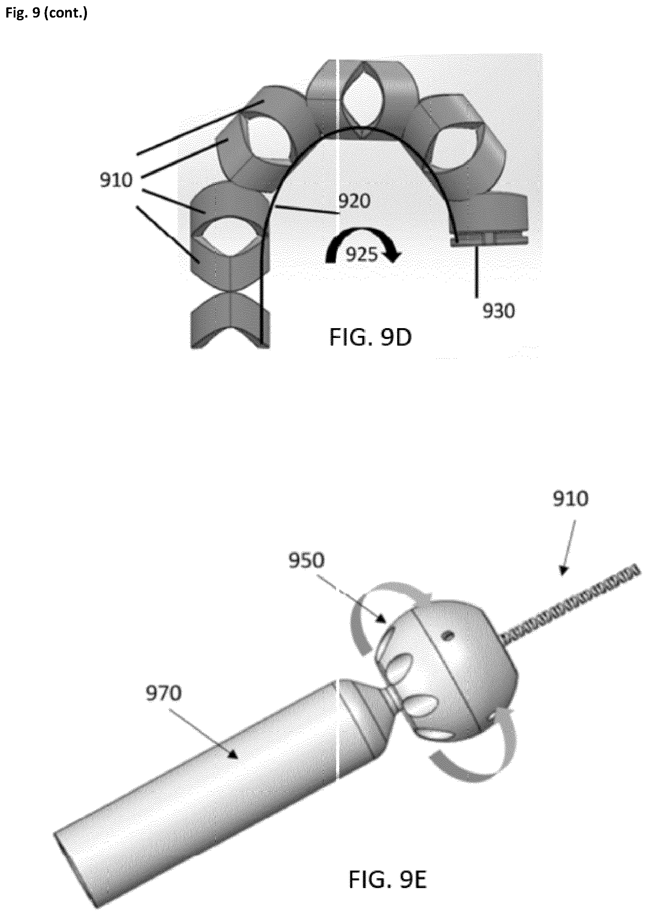

Fig. 9D shows a side view of a steering mechanism when one of the cables has been retracted, such that the steering mechanism is steered in the direction illustrated by arrow 925.

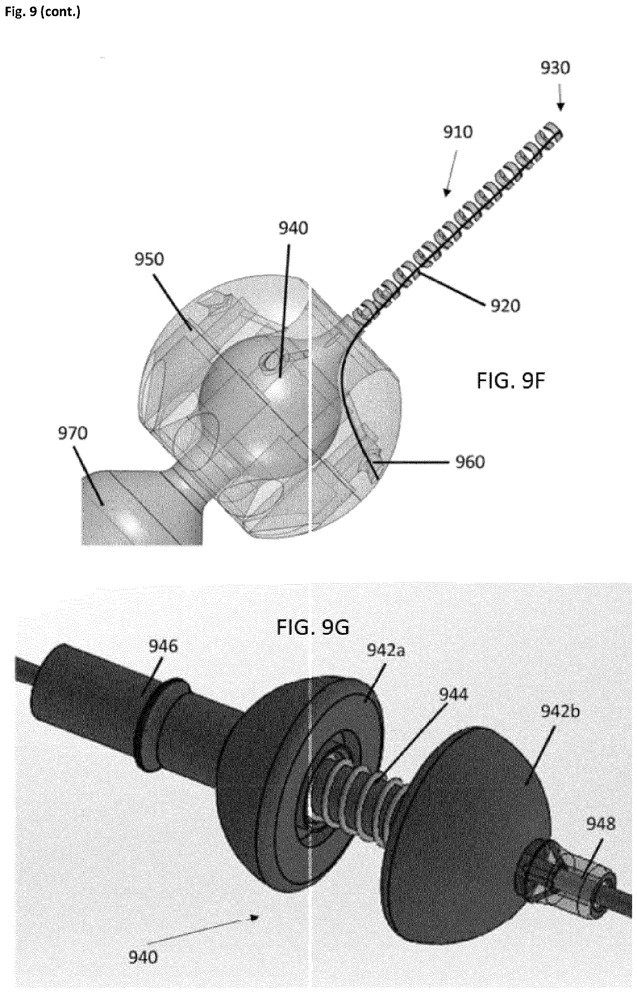

Fig. 9E shows a perspective view of the third actuator according to some embodiments. Fig. 9F shows a perspective view of the third actuator shown in Fig. 9E with the outer shell 950 depicted as transparent. The third actuator comprises an inner element 940 and an outer shell 950 housing the inner element 940. Each of the at least one cables 920 are fixed to the outer shell at a proximal portion 960, for example by welding, adhesive, or about a fixed winch. The inner element 940 is fixedly connected to the tube wall 910 either directly or indirectly via one or more intermediate components. The outer shell 950 is rotatable relative to the inner element 940. Optionally, a handle 970 may be fixed to the inner element 940 through the outer shell 950. Rotating the outer shell 950 about the inner element 940 causes one of the cables 920 to be retracted, which steers the tube wall in a certain direction as discussed above. The body may extend through the handle 970 and the inner element 940 such that it may be advanced through the steering mechanism.

Fig. 9G shows a perspective view of the inner element according to some embodiments. The inner element 940 may comprise one or more components which are biased to abut the outer shell 950. For example, the inner element 940 may comprise two hemispheres 942a, 942b, which are biased away from each other to abut the inner surface of the outer shell 950. The biasing causes a frictional fit to be obtained between the inner element 940 and outer shell 950, such that they may only slide relative to each other when a sufficient force is applied, meaning that once a desired direction of the steering mechanism is achieved, it may be maintained in position by the third actuator. The inner element may be connected to the handle via a handle connector 946, and may be connected to the tube wall via an intermediate portion 948. The intermediate portion 948 may be incompressible and the cables 920 may be threaded through the intermediate portion and to the tube wall.

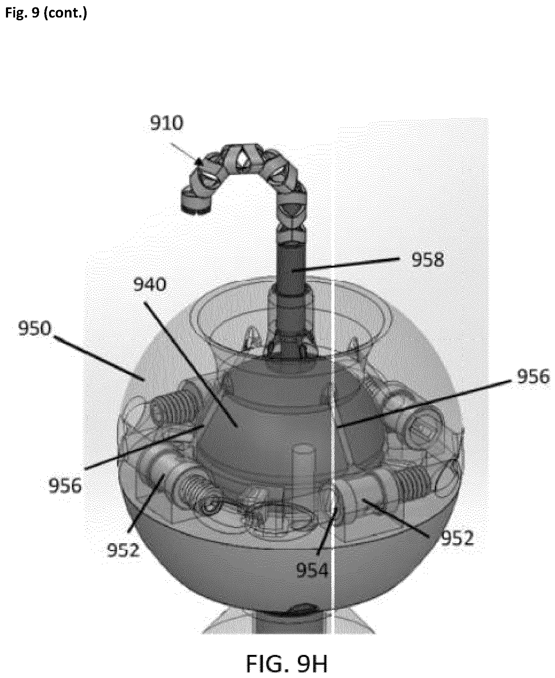

Fig. 9H shows a perspective view of a steering mechanism according to some embodiments, with the outer shell 950 illustrated as partially transparent. The steering mechanism of Fig. 9H also comprises an inner element 940 and an outer shell 950 housing the inner element 940 and rotatable relative to inner element 940, as described above. The steering mechanism may also comprise the handle 970. Further, each of the cables 920 are fixedly connected to the outer shell via a winch 952 (i.e. the cables are wound to the winch). The winch may be wound or unwound using an adjuster 954 (for example a flat head adjuster) which is accessible externally to the outer shell 950, meaning that the default tensions for each cable 920 may be calibrated after assembly of the steering mechanism. The rings 910 forming the tube wall may be fixedly connected to the inner element 940 via an inflexible tubular intermediate 958. The tubular intermediate 958 may be of any suitable length. For example, in embodiments where the outer catheter is used, the tubular intermediate 958 may be configured to extend through any inflexible parts of the outer catheter.

The cables 920 may extend through channels 956 formed in the inner surface of outer shell 950, and thus avoid contact with the inner element 940, avoiding damage to the cables as the outer shell rotates relative to the inner shell 940. The cables 920 may extend from the channels 956 and through lumens formed in the wall of the tubular intermediate 958, and to the tube wall.

The outer shell 950, inner element 940, handle 970, winches 952 and tubular intermediate 958 may be made of medical grade injection moldable or 3D printed plastic resin such as ABS, PET, DELRIN or PTFE.

The steering mechanism may instead be electronically controlled. For example, the steering mechanism shown in Fig. 9H may comprise an electronic controller fixed to the inner element 940, and comprising motors for actuating movement of the outer shell 950 relative to the inner element 940. Alternatively, a steering mechanism may comprise a plurality of connected rings forming a tube wall configured to at least partially surround a portion of the body, and at least two opposing cables extending longitudinally through the tube wall and attached to a distal end of the tube wall, wherein the cables are connected to a motor configured to contract the cables to steer the steering mechanism.

The tube wall of the steering mechanism is sized to slidably receive the body 110. The outer diameter of the tube wall may be about 1.4mm to 1.8mm or about 1.5mm to 1.7mm, for example 1.6mm. In embodiments where the steering mechanism is used, the body 110 may be configured to advance beyond the end of the steering mechanism by about 20mm to 40mm, for example about 30mm.

A proximal end of the steering mechanism may be configured to connect with a distal end of the speculum lock 700a or 700b. For example, the steering mechanism may comprise a connecting mechanism configured to connect with a corresponding connecting mechanism on the speculum lock 700a or 700b, so that the guide 705 and the steering mechanism define a continuous lumen through which body 110 slidably extends.

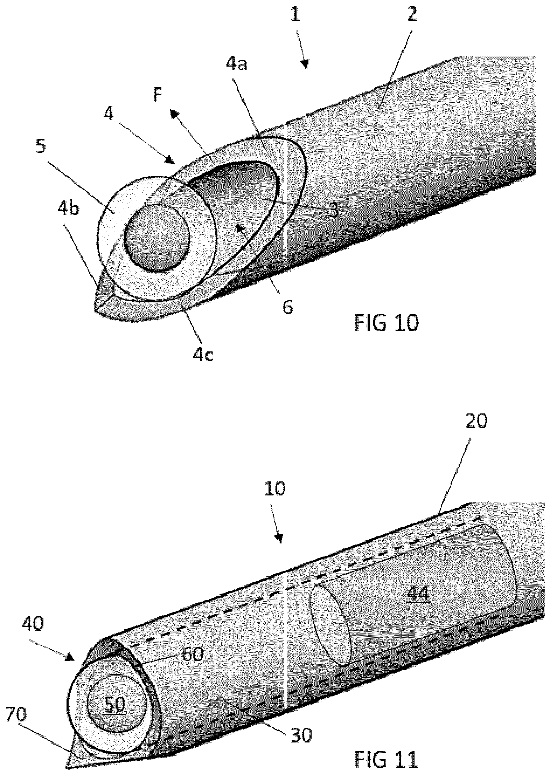

Fig. 10 shows a perspective view of an apparatus 1 comprising an elongated body which may be the inner body 160 in any of the disclosed embodiments herein. The apparatus 1 comprises an elongated body 2 (for example a needle) comprising a distal tip 4 and a lumen 3 extending therethrough to an aperture 6 at the distal tip 4. The lumen 3 is configured to receive and expel a fertilized egg or embryo 5 (hereinafter referred to as "egg" for brevity) through the aperture 6. A plunger (not shown) is slidably received within the lumen 3. The distal tip 4 is configured to pierce the maternal uterine endometrium and comprises a first surface 4a which is bevelled with respect to the longitudinal axis of the elongated body 2. The distal tip 4 further comprises a piercing portion at its most distal end, which is a double-bevelled piercing portion comprising first and second surfaces 4b and 4c which are bevelled with respect to the first surface 4a to form a sharp point at the distal end of the distal tip 4. In use, the plunger is retractable in a proximal direction opposite to the distal tip 4, which creates suction at the distal tip 4 and can be used to place the egg 5 within the lumen 3. The egg 5 is typically placed in the lumen with fluid proximal to the egg 5 within the lumen 3. In order to implant the egg 5 into the endometrium, the elongated body 2 penetrates the endometrium using the piercing distal tip and the plunger is advanced distally to expel the fluid and egg 5 within the lumen 3. The egg 5 is generally pushed distally due to the pressure transmitted from the plunger via the fluid. However, when the egg 5 reaches the most distal part of the elongated body 2, a gap is formed in the aperture 6 as shown in Fig. 10, meaning that the fluid contained in the lumen 3 can escape the lumen via fluid pathway F such that the fluid is expelled without exerting a pressure on the egg 5. Therefore, in some cases, the egg 5 can remain attached to the distal tip 4 even after the plunger is fully advanced and the fluid has been fully expelled. This reduces the change of successful implantation as the egg 5 may be dislodged or even removed from the endometrium as the elongated body 2 is removed.

Fig. 11 shows a schematic perspective view of an apparatus 10 suitable for delivering a fertilized egg or embryo 50 (hereinafter referred to as "egg" for brevity) into a maternal uterine endometrium according to one or more embodiments. The apparatus 10 comprises an elongated body 20 comprising a distal tip 40 and a lumen 30 extending therethrough to an aperture 60 at the distal tip 40. The elongated body 20 may be the inner body 160 in any of the disclosed embodiments herein. The lumen 30 is configured to receive and expel the egg 50 through the aperture 60. The apparatus 10 also comprises a plunger 44 configured to be slidably received within the lumen 30. The distal tip 40 comprises a piercing portion 70 for piercing the maternal uterine endometrium, and the aperture 60 has a concave shape. The piercing portion may take any suitable shape and sharpness which is suitable for piercing the endometrium. In some embodiments, the piercing portion may comprise a point or edge having an edge radius of less than 2µm, preferably less than 1µm. The concave shape of the aperture 60 reduces or eliminates the amount of fluid which can escape the lumen 30 without exerting pressure on the egg 50 at the distal end of the distal tip 40, such that the egg 50 is less likely to remain on the distal tip 40 when the fluid is fully expelled. This increases the likelihood of successful implantation.

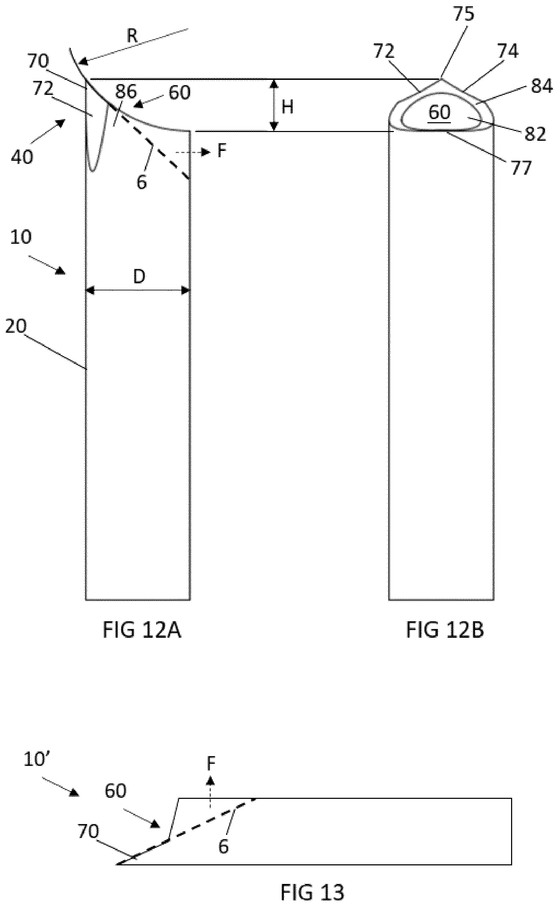

Figs. 12A and 12B show different schematic side views of the apparatus 10 shown in Fig. 11. In Fig. 12A, the concave shape of the aperture 60 is shown against the shape of the aperture 6 of the apparatus 1. It can be seen that the available fluid pathway F shown in Fig. 10 is prevented by the concave shape of the aperture 60. As shown in Fig. 12B, the piercing portion 70 may comprise a double-bevelled tip, comprising back cut bevels, for piercing the maternal endometrium. In particular, the piercing portion 70 may comprise first and second surfaces 72, 74, which are bevelled with respect to the outer surface of the elongated body 20, the bevelled surfaces 72 and 74 forming a sharp point 75 at the distal end of the distal tip 40.

As shown in Figs. 12A and 12B, the distally facing surface 84 of the distal tip 40 which defines the aperture 60 may have a curved concave shape having a radius of curvature R, so that the aperture 60 has a curved concave shape having a radius of curvature R. A curved concave shape having a radius of curvature is preferred because the aperture 60 generally follows the spherical shape of the egg 50, such that when the egg 50 is at the distal end of the distal tip 40, the aperture conforms to the shape of the egg 50 and prevents any fluid pathways F from occurring. In preferred embodiments the radius of curvature R is between 1.5 and 4 times the outer diameter D of the elongated body 20, which is considered optimal for preventing fluid pathways F from occurring for typical egg and embryo sizes.

As shown in Fig. 12A, preferably the curved concave shape extends between the piercing portion 70 and a lowermost portion of the distal tip 40, wherein the lowermost portion of the distal tip 40 extends perpendicularly to the longitudinal axis of the elongated body 20. This ensures that the fluid pathways are minimised whilst also maintaining contact between the distal tip 40 and the egg 50 when it is positioned at the distal end of the elongated body 20.

In preferred embodiments, the height of the distal tip 40 as measured from the lowermost portion 77 of the distal tip 40 to the distal end 75 of the piercing portion 70 is between 0.25 and 1.25 times the outer diameter D of the elongated body 20, and more preferably between 0.5 and 1.25 times the outer diameter D of the elongated body 20.

The distal tip may be made of a biocompatible metal, for example stainless steel.

In preferred embodiments, the distal tip 40 comprises a hydrophobic coating. The hydrophobic coating may be provided on one or more of an inner surface 82 of the distal tip 40, an outer surface 86 of the distal tip 40 and a distally facing surface 84 of the distal tip 40. The hydrophobic coating may comprise or consist of a polymer or polymer composite, preferably any of the following: parylene, acrylic, polyethylene, polyurethane and polytetrafluorethylene and composites thereof. The hydrophobic coating further reduces the likelihood of the egg 50 remaining on the distal tip 40 after the fluid has been expelled.

Fig. 13 shows a schematic side view of another apparatus 10' according to one or more embodiments. The apparatus may comprise any of the features disclosed with reference to Figs. 11, 12A and 12B, except the aperture 60 of apparatus 10' does not comprise a curved concave shape. Instead of the curved concave shape shown in Figs. 11, 12A and 11B, the aperture 60 may take a concave shape formed by a plurality of planar surfaces. It will be appreciated that while in the illustrated embodiment the concave shape is formed by two planar surfaces, it may be formed by any number of a plurality of planar surfaces. The concave shape of the aperture 60 is again shown against the shape of the aperture 6 of the apparatus 1. It can be seen that the available fluid pathway F shown in Fig. 10 is prevented by the concave shape of the aperture 60 of apparatus 10'.

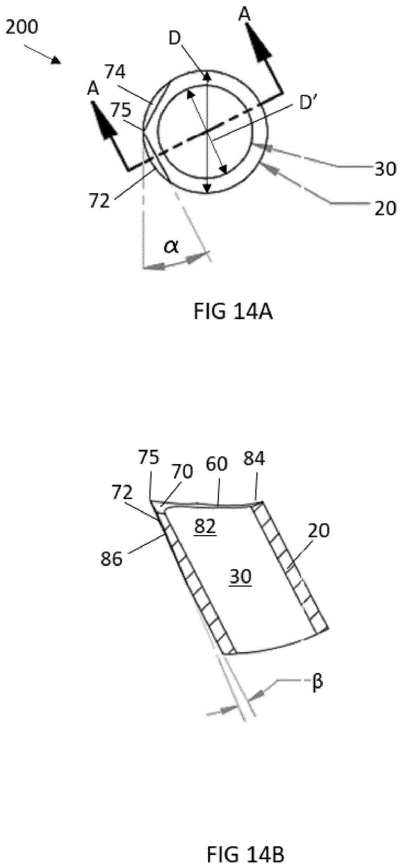

Fig. 14A shows a schematic top view of an apparatus 200 according to one or more embodiments. Fig. 14B shows a cross-sectional side view of the apparatus 200 shown in Fig. 14A taken along the line A-A. The apparatus 200 may comprise any of the features of the apparatuses described herein (for example apparatuses 10 or 10'). The piercing portion 70 first and second surfaces 72, 74, which are bevelled with respect to the outer surface of the elongated body 20, the bevelled surfaces 72 and 74 forming a sharp point 75 (a double-bevelled tip) at the distal end of the distal tip 40. The bevelled surface 72 is defined by bevel angle a in the plane perpendicular to the longitudinal axis of the elongated body 20 and draft angle β with respect to the longitudinal axis of the elongated body 20. The bevel angle a is defined as the angle that the plane containing the bevelled surface 72 makes with the tangent of the surface of the elongated body 20 at the point 75. The draft angle β is defined as the angle that the plane containing the bevelled surface 72 makes with the longitudinal axis of the elongated body 20. The bevel angle and the draft angle of the bevelled surface 74 are similarly defined. The bevel angles of the bevelled surfaces 72 and 74 are preferably between 20° and 40°. The draft angles of the bevelled surfaces 72 and 74 are preferably between 1° and 20°. The bevel and draft angles of the bevelled surfaces 72 and 74 may be the same (as illustrated) or different.

In preferred embodiments, the diameter of the lumen 30 is between 1 and 2 times the outer diameter of the egg to be implanted, more preferably between 1 and 1.5 times the outer diameter of the egg to be implanted.

In preferred embodiments, outer diameter of the elongated body 20 is between 1.5 and 3 times the diameter of the lumen 30., more preferably between 1.5 and 2 times the diameter of the lumen 30.

## Claims

1. An apparatus (100) suitable for delivering a fertilized egg or embryo into a maternal uterine endometrium in humans or any other mammalian species, comprising a body (110) configured to fit within a lumen of the female reproductive system, the body comprising:
one or more lumens extending from a proximal end of the body (110) to a distal portion of the body (110) and having a distal opening at the distal portion of the body (110), wherein a first lumen (120) of the one or more lumens slidably receives an inner body (160) having a distal end suitable for penetrating the endometrial epithelium, the inner body (160) comprising an inner lumen for receiving a fertilized egg;
an actuator (195) operable to expel the fertilized egg from the inner lumen; and
a covering layer (170a) at least partially covering the distal opening of at least one lumen of the one or more lumens;
wherein the covering layer (170a) at least partially covers the distal opening of the first lumen (120) and
**characterised in that** the inner body (160) is configured to perforate through the covering layer (170a) when the inner body (160) is advanced out from the distal opening of the body.

2. The apparatus (100) of claim 1, further comprising a measurement assembly, the measurement assembly extending through a lumen of the one or more lumens and comprising a measurement portion (170) proximal a distal opening of the one or more lumens, the measurement portion (170) configured to measure whether the apparatus (100) is in a first state indicating that a distance between the distal end of the body (110) or the inner body (160) and the endometrial epithelium is greater than a predetermined distance, or a second state indicating that a distance between the distal end of the body (110) or the inner body (160) and the endometrial epithelium is equal to or less than a predetermined distance; wherein the measurement portion (170) is at least partially covered by the covering layer (170a).

3. The apparatus (100) of claim 2, wherein the measurement assembly comprises a capacitance sensor configured to make electrical contact with a distal portion of the inner body (160) which is electrically conductive, and to provide an indication of capacitance, wherein the first state is indicated by a capacitance measurement below a threshold value and the second state is indicated by a capacitance measurement above the threshold value.

4. The apparatus (100) of any of claims 1 to 3, further comprising a camera (171) proximal the distal opening and configured to view a portion of the endometrial epithelium.

5. The apparatus (100) of claim 4, wherein the covering layer (170a) covers the camera (171) so that the camera (171) is not exposed to mucus when the distal portion of the apparatus (100) is placed in the lumen of the female reproductive system.

6. The apparatus (100) of any of claims 1 to 5, wherein the covering layer (170a) comprises a biocompatible transparent or semi-transparent film, preferably wherein the covering layer (170a) is a transparent or semi-transparent film comprising or consisting of any of the following: polyolefin, PVC, paraffin, cellulose and derivates, hyaluronic acid thin films, parylene C-films, collagen, gelatin, vegetable casings, alginates and composites thereof.

7. A system (101) comprising the apparatus (100) of any of claims 1 to 6 and a device (700a, 700b, 700c) for engaging a speculum, comprising:
two or more expandable engaging elements (710) for outwardly engaging a speculum to fix the device (700a, 700b, 700c) to the speculum, wherein the two or more expandable engaging elements (710) comprise a plurality of expanded configurations for engaging a plurality of speculum sizes; and
a guide (705) for guiding the body (110) between the expandable engaging elements (710) and through the speculum when the device (700a, 700b, 700c) is fixed to the speculum.

8. The system (101) of claim 7, wherein the guide (705) at least partially conforms to an outer surface of the body (110) to allow longitudinal movement of the body (110) through the guide (705) and to prevent lateral movement of the body (110) in the guide (705).

9. The system (101) of claim 7 or 8, further comprising a first and second handle (762a, 762b) connected to a first and second engaging element (710) respectively, the handles pivotably connected to one another such that movement of the handles (762a, 762b) in a direction about the pivot (761) causes the engaging elements (710) to move apart to the expanded configurations.

10. The system (101) of any of claims 7 to 9, further comprising a guide locking mechanism (767) having a first configuration in which the guide locking mechanism (767) is disengaged with the guide (705), and a second configuration in which guide locking mechanism (767) grips the guide (705) and provides a compressive force to the guide (705) to inhibit longitudinal movement of the body (110) through the guide (705).

11. The system (101) of any of claims 7 to 10 wherein an inner surface of the guide (705) is at least partially coated with a frictional coating having a higher coefficient of friction than the inner surface of the guide (705) and/or wherein one or more surfaces of the engaging elements (710) configured to engage the speculum are at least partially coated with a frictional coating having a higher coefficient of friction than said outer surface.

12. The apparatus (100) or system (101) of any preceding claim, further comprising:
a first actuator (190) operable to advance the inner body (160) out from the distal opening of the body (110); and
a second actuator (195) operable to expel a fertilized egg from an inner lumen of the inner body (160);
wherein the inner body (160) comprises a hydrophobic coating formed on a distal portion of the inner body (160).

13. The apparatus (100) or system (101) of claim 12, wherein the hydrophobic coating is provided on at least a distal portion of an inner surface of the inner body (160) defining the inner lumen.

14. The apparatus (100) or system (101) of claim 12 or 13, wherein the hydrophobic coating is provided on an outer surface and/or the distal tip of the inner body (160).

15. The apparatus (100) or system (101) of any of claims 12 to 14, wherein the inner body (160) comprises a bevelled tip (163).

## Patentansprüche

1. Gerät (100), welches dafür geeignet ist, ein befruchtetes Ei oder einen Embryo in ein mütterliches Endometrium in Menschen oder einer anderen Säugetierart abzugeben, umfassend einen Körper (110), welcher dazu ausgebildet ist, innerhalb eines Lumens des weiblichen Fortpflanzungssystems einzupassen, wobei der Körper Folgendes umfasst:
ein oder mehrere Lumina, welche sich von einem proximalen Ende des Körpers (110) bis zu einem distalen Teil des Körpers (110) erstrecken und eine distale Öffnung am distalen Teil des Körpers (110) aufweisen, wobei ein erstes Lumen (120) des einen oder der mehreren Lumina einen inneren Körper (160) gleitend aufnimmt, welcher ein distales Ende aufweist, welches dafür geeignet ist, in das Endometriumepithel einzudringen, wobei der innere Körper (160) ein inneres Lumen zum Aufnehmen eines befruchteten Eis umfasst;
ein Stellglied (195), welches zum Ausstoßen des befruchteten Eis aus dem inneren Lumen betrieben werden kann; und
eine Deckschicht (170a), welche mindestens teilweise die distale Öffnung mindestens eines Lumens des einen oder der mehreren Lumina deckt;
wobei die Deckschicht (170a) mindestens teilweise die distale Öffnung des ersten Lumens (120) deckt und
**dadurch gekennzeichnet, dass** der innere Körper (160) dazu ausgebildet ist, die Deckschicht (170a) zu durchbohren, wenn der innere Körper (160) von der distalen Öffnung des Körpers raus vorgerückt wird.

2. Gerät (100) nach Anspruch 1, zusätzlich umfassend eine Messbaugruppe, wobei sich die Messbaugruppe durch ein Lumen des einen oder der mehreren Lumina erstreckt und einen Messteil (170) umfasst, welcher proximal zu einer distalen Öffnung des einen oder der mehreren Lumina ist, wobei der Messteil (170) dazu ausgebildet ist, zu messen, ob sich das Gerät (100) in einem ersten Zustand, welcher andeutet, dass ein Abstand zwischen dem distalen Ende des Körpers (110) oder dem inneren Körper (160) und dem Endometriumepithel größer als ein vorbestimmter Abstand ist, oder einem zweiten Zustand, welcher andeutet, dass ein Abstand zwischen dem distalen Ende des Körpers (110) oder dem inneren Körper (160) und dem Endometriumepithel gleich oder kleiner als ein vorbestimmter Abstand ist, befindet; wobei der Messteil (170) mindestens teilweise von der Deckschicht (170a) gedeckt ist.

3. Gerät (100) nach Anspruch 2, wobei die Messbaugruppe einen Kapazitätssensor umfasst, welcher dazu ausgebildet ist, mit einem distalen Teil des inneren Körpers (160), welcher elektrisch leitfähig ist, einen elektrischen Kontakt herzustellen, und eine Kapazitätsangabe bereitzustellen, wobei der erste Zustand durch eine Kapazitätsmessung unter einem Schwellenwert angedeutet wird und der zweite Zustand durch eine Kapazitätsmessung über dem Schwellenwert angedeutet wird.

4. Gerät (100) nach einem der Ansprüche 1 bis 3, zusätzlich umfassend eine Kamera (171), welche proximal zur distalen Öffnung ist und dazu ausgebildet ist, einen Teil des Endometriumepithels zu betrachten.

5. Gerät (100) nach Anspruch 4, wobei die Deckschicht (170a) die Kamera (171) deckt, sodass die Kamera (171) nicht Schleim ausgesetzt ist, wenn der distale Teil des Geräts (100) im Lumen des weiblichen Fortpflanzungssystems platziert ist.

6. Gerät (100) nach einem der Ansprüche 1 bis 5, wobei die Deckschicht (170a) einen biokompatiblen durchsichtigen oder halbdurchsichtigen Film umfasst, wobei vorzugsweise die Deckschicht (170a) ein durchsichtiger oder halbdurchsichtiger Film ist, welcher eins der Folgenden umfasst oder daraus besteht: Polyolefin, PVC, Paraffin, Cellulose und Derivaten, Hyaluronsäure-Dünnschichten, Parylene C-Filmen, Collagen, Gelatine, pflanzlichen Hüllen, Alginaten und Verbunden davon.

7. System (101) umfassend das Gerät (100) nach einem der Ansprüche 1 bis 6 und eine Vorrichtung (700a, 700b, 700c) zum Eingreifen eines Spekulums, umfassend:
zwei oder mehr expandierbare Eingriffselemente (710) zum äußerlichen Eingreifen eines Spekulums, um die Vorrichtung (700a, 700b, 700c) am Spekulum zu fixieren, wobei die zwei oder mehr expandierbaren Eingriffselemente (710) eine Vielzahl von expandierten Ausbildungen zum Eingreifen einer Vielzahl von Spekulumgrößen umfassen; und
eine Führung (705) zum Führen des Körpers (110) zwischen den expandierbaren Eingriffselementen (710) und durch das Spekulum, wenn die Vorrichtung (700a, 700b, 700c) am Spekulum fixiert ist.

8. System (101) nach Anspruch 7, wobei sich die Führung (705) mindestens teilweise an eine äußeren Oberfläche des Körpers (110) anpasst, um eine Längsbewegung des Körpers (110) durch die Führung (705) zu erlauben und eine seitliche Bewegung des Körpers (110) in der Führung (705) zu verhindern.

9. System (101) nach Anspruch 7 oder 8, zusätzlich umfassend einen ersten und einen zweiten Griff (762a, 762b), welche jeweils mit einem ersten und einem zweiten Eingriffselement (710) verbunden sind, wobei die Griffe miteinander schwenkbar verbunden sind, sodass eine Bewegung der Griffe (762a, 762b) in einer Richtung um das Schwenkelement (761) herum bewirkt, dass sich die Eingriffselemente (710) zu expandierten Ausbildungen voneinander wegbewegen.

10. System (101) nach einem der Ansprüche 7 bis 9, zusätzlich umfassend einen Führungssperrmechanismus (767), welcher eine erste Ausbildung, in welcher der Führungssperrmechanismus (767) mit der Führung (705) außer Eingriff ist, und eine zweite Ausbildung, in welcher der Führungssperrmechanismus (767) die Führung (705) greift und eine Druckkraft in Bezug auf die Führung (705) bereitstellt, um eine Längsbewegung des Körpers (110) durch die Führung (705) zu hemmen, aufweist.

11. System (101) nach einem der Ansprüche 7 bis 10, wobei eine innere Oberfläche der Führung (705) mindestens teilweise mit einer Reibbeschichtung beschichtet ist, welche einen höheren Reibungskoeffizient als die innere Oberfläche der Führung (705) aufweist, und/oder wobei eine oder mehrere Oberflächen der Eingriffselemente (710), welche dazu ausgebildet sind, mit dem Spekulum einzugreifen, mindestens teilweise mit einer Reibbeschichtung beschichtet sind, welche einen höheren Reibungskoeffizient als die genannte äußere Oberfläche aufweist.

12. Gerät (100) oder System (101) nach einem vorhergehenden Anspruch, zusätzlich umfassend:
ein erstes Stellglied (190), welches zum Vorrücken des inneren Körpers (160) von der distalen Öffnung des Körpers (110) raus betrieben werden kann; und
ein zweites Stellglied (195), welches zum Ausstoßen eines befruchteten Eis aus einem inneren Lumen des inneren Körpers (160) betrieben werden kann;
wobei der innere Körper (160) eine hydrophobe Beschichtung umfasst, welche auf einem distalen Teil des inneren Körpers (160) gebildet ist.

13. Gerät (100) oder System (101) nach Anspruch 12, wobei die hydrophobe Beschichtung auf mindestens einem distalen Teil einer inneren Oberfläche des inneren Körpers (160), welche das innere Lumen unter definiert, vorgesehen ist.

14. Gerät (100) oder System (101) nach Anspruch 12 oder 13, wobei die hydrophobe Beschichtung auf einer äußeren Oberfläche und/oder der distalen Spitze des inneren Körpers (160) vorgesehen ist.

15. Gerät (100) oder System (101) nach einem der Ansprüche 12 bis 14, wobei der innere Körper (160) eine abgeschrägte Spitze (163) umfasst.

## Revendications

1. Appareil (100) approprié pour administrer un ovule fécondé ou embryon dans un endomètre utérin maternel chez des humains ou toute autre espèce de mammifère, comprenant un corps (110) configuré pour s'insérer dans une lumière de l'appareil reproducteur féminin, le corps comprenant :
une ou plusieurs lumières s'étendant d'une extrémité proximale du corps (110) jusqu'à une portion distale du corps (110) et présentant une ouverture distale dans la portion distale du corps (110), dans lequel une première lumière (120) de l'une ou plusieurs lumières reçoit de manière coulissante un corps interne (160) ayant une extrémité distale appropriée pour pénétrer dans l'épithélium endométrial, le corps interne (160) comprenant une lumière interne pour recevoir un ovule fécondé ;
un actionneur (195) pouvant être actionné pour expulser l'ovule fécondé à partir de la lumière interne ; et
une couche de recouvrement (170a) recouvrant au moins partiellement l'ouverture distale d'au moins une lumière de l'une ou plusieurs lumières ;
dans lequel la couche de recouvrement (170a) recouvre au moins partiellement l'ouverture distale de la première lumière (120) et
**caractérisé en ce que** le corps interne (160) est configuré pour perforer à travers la couche de recouvrement (170a) lorsqu'on fait avancer le corps interne (160) à partir de l'ouverture distale du corps.

2. Appareil (100) selon la revendication 1, comprenant en outre un ensemble de mesure, l'ensemble de mesure s'étendant à travers une lumière de l'une ou plusieurs lumières et comprenant une portion de mesure (170) proximale à une ouverture distale de l'une ou plusieurs lumières, la portion de mesure (170) étant configurée pour mesurer si l'appareil (100) se trouve dans un premier état indiquant qu'une distance entre l'extrémité distale du corps (110) ou du corps interne (160) et l'épithélium endométrial est supérieure à une distance prédéterminée, ou un deuxième état indiquant qu'une distance entre l'extrémité distale du corps (110) ou du corps interne (160) et l'épithélium endométrial est égale ou inférieure à une distance prédéterminée; dans lequel la portion de mesure (170) est au moins partiellement recouverte par la couche de recouvrement (170a).

3. Appareil (100) selon la revendication 2, dans lequel l'ensemble de mesure comprend un capteur de capacité configuré pour établir un contact électrique avec une portion distale du corps interne (160) qui est électriquement conductrice, et pour fournir une indication de la capacité, dans lequel le premier état est indiqué par une mesure de capacité inférieure à une valeur seuil et le deuxième état est indiqué par une mesure de capacité supérieure au valeur seuil.

4. Appareil (100) selon l'une quelconque des revendications 1 à 3, comprenant en outre une caméra (171) proximale à l'ouverture distale et configurée pour visualiser une portion de l'épithélium endométrial.

5. Appareil (100) selon la revendication 4, dans lequel la couche de recouvrement (170a) recouvre la caméra (171) de telle sorte que la caméra (171) n'est pas exposée à mucosité lorsque la portion distale de l'appareil (100) est placée dans la lumière de l'appareil reproducteur féminin.

6. Appareil (100) selon l'une quelconque des revendications 1 à 5, dans lequel la couche de recouvrement (170a) comprend un film biocompatible transparent ou semi-transparent, de préférence dans lequel la couche de recouvrement (170a) est un film transparent ou semi-transparent comprenant ou constitué de l'un des éléments suivants : polyoléfine, PVC, paraffine, cellulose et dérivés, films minces d'acide hyaluronique, films de parylène C, collagène, gélatine, boyaux végétaux, alginates et composites ceux-ci.

7. Système (101) comprenant l'appareil (100) selon l'une quelconque des revendications 1 à 6 et un dispositif (700a, 700b, 700c) pour engager un spéculum, comprenant :
deux ou plusieurs éléments d'engagement expansibles (710) pour engager vers l'extérieur un spéculum pour fixer le dispositif (700a, 700b, 700c) au spéculum, dans lequel les deux ou plusieurs éléments d'engagement expansibles (710) comprennent une pluralité de configurations étendues pour engager une pluralité de tailles de spéculum ; et
un guide (705) pour guider le corps (110) entre les éléments d'engagement expansibles (710) et à travers le spéculum lorsque le dispositif (700a, 700b, 700c) est fixé au spéculum.

8. Système (101) selon la revendication 7, dans lequel le guide (705) épouse au moins partiellement une surface externe du corps (110) pour permettre le mouvement longitudinal du corps (110) à travers le guide (705) et pour empêcher le mouvement latéral du corps (110) dans le guide (705).

9. Système (101) selon la revendication 7 ou 8, comprenant en outre une première et une deuxième poignées (762a, 762b) reliées à un premier et un deuxième éléments d'engagement (710), respectivement, les poignées étant reliées de manière pivotante l'une à l'autre de telle sorte que le mouvement des poignées (762a, 762b) dans une direction autour du pivot (761) provoque l'écartement des éléments d'engagement (710) vers les configurations étendues.

10. Système (101) selon l'une quelconque des revendications 7 à 9, comprenant en outre un mécanisme de verrouillage de guide (767) présentant une première configuration dans laquelle le mécanisme de verrouillage de guide (767) est désengagé du guide (705), et une deuxième configuration dans laquelle le mécanisme de verrouillage de guide (767) serre le guide (705) et fourni une force de compression au guide (705) pour empêcher le mouvement longitudinal du corps (110) à travers le guide (705).

11. Système (101) selon l'une quelconque des revendications 7 à 10, dans lequel une surface interne du guide (705) est au moins partiellement revêtue avec un revêtement de friction ayant un coefficient de friction supérieur à la surface interne du guide (705) et/ou dans lequel une ou plusieurs surfaces des éléments d'engagement (710) configurés pour engager le spéculum sont au moins partiellement revêtues avec un revêtement de friction ayant un coefficient de friction supérieur à ladite surface externe.

12. Appareil (100) ou système (101) selon l'une quelconque des revendications précédentes, comprenant en outre :
un premier actionneur (190) pouvant être actionné pour faire avancer le corps interne (160) à partir de l'ouverture distale du corps (110) ; et
un deuxième actionneur (195) pouvant être actionné pour expulser un ovule fécondé à partir d'une lumière interne du corps interne (160) ;
dans lesquels le corps interne (160) comprend un revêtement hydrophobe formé sur une portion distale du corps interne (160).

13. Appareil (100) ou système (101) selon la revendication 12, dans lesquels le revêtement hydrophobe est prévu sur au moins une portion distale d'une surface interne du corps interne (160) définissant la lumière interne.

14. Appareil (100) ou système (101) selon la revendication 12 ou 13, dans lesquels le revêtement hydrophobe est prévu sur une surface externe et/ou la pointe distale du corps interne (160).

15. Appareil (100) ou système (101) selon l'une quelconque des revendications 12 à 14, dans lesquels le corps interne (160) comprend une pointe biseautée (163).
